# EUROPEAN PATENT SPECIFICATION

(11) **EP 1 403 250 B1**
(45) Date of publication and mention of the grant of the patent: **18.07.2007**
(21) Application number: 02736191.4
(22) Date of filing: 27.06.2002
(51) Int. Cl.: C07D 213/74, C07D 401/14, C07D 405/14, C07D 409/14, C07D 487/04, A61K 31/551, A61K 31/44, A61P 11/06, A61P 37/08, A61P 29/00, A61P 9/10, A61P 43/00, C07D 241/00

(54) **BIS(5-ARYL-2-PYRIDYL) DERIVATIVE**
BIS(5-ARYL-2-PYRIDYL)DERIVATE
DERIVE DE BIS(5-ARYL-2-PYRIDYLE)

(30) Priority: 29.06.2001 US 893698
(43) Date of publication of application: 31.03.2004
(73) Proprietor: Kowa Co., Ltd., Nagoya-shi, Aichi 460-8625 (JP)
(72) Inventor: ISHIWATA, Hiroyuki, ICHIKAWA-SHI, Chiba 272-0824 (JP); SATO, Seiichi, SUGINAMI-KU, Tokyo 167-0043 (JP); KABEYA, Mototsugu, HIGASHIMURAYAMA-SHI, Tokyo 189-0022 (JP); ODA, Soichi, Nishikasugai-gun Aichi 452-0055 (JP); SUDA, Makoto, Tokyo 189-0022 (JP); SHIBASAKI, Manabu, CHIBA-SHI, Chiba 260-0851 (JP)
(74) Representative: Hartz, Nikolai
(86) International application number: PCT/JP2002/006492
(87) International publication number: WO 2003/002537

(56) References cited:
- EP-A1- 0 926 138
- EP-A1- 1 052 238
- WO-A1-98/18782

## Description

### Technical Field

This invention relates to novel bis(5-aryl-2-pyridyl) derivatives or salts thereof, and also to medicines which comprise the bis(5-aryl-2-pyridyl) derivatives or salts thereof as active ingredients and are useful for the prevention or treatment of allergic immune diseases.

### Background Art

IgE, a class of immunoglobulin (Ig), is a allergen-specific molecule produced by IgE producing cells differentiated from B cells, when triggered by contact of immunocompetent cells with an allergen in the body.

IgE is produced in a target organ of allergy, and binds to a receptor on surfaces of mast cells or basophils as principal effector cells in an allergic reaction (sensitized state). From the mast cells stimulated as a result of intrusion of the allergen into the body after the sensitization and its reaction with the specific IgE, histamine, a leucotriene, a prostaglandin, an allergic chemical transmitter such as PAF, or a detrimental enzyme such as tryptase is released, and an immediate-response allergic reaction such as increased vasopermeability, smooth muscle constriction or vasodilation is provoked. From the stimulated mast cells, a cytokine capable of directly activating other immune system cells, such as IL-4, is also secreted. As a result, eosinophils, basophils or the like infiltrate tissues, and an allergic chemical transmitter or a histodetrimental protein such as MBP, which is secreted by these inflammatory cells, induces a delayed allergic reaction and protracts and worsen an allergic symptom.

Hence, the abnormal production of IgE is associated deeply with various allergic immune diseases, such as asthma, atopic dermatitis, allergic rhinitis, inflammatory colitis, contact-type dermatitis and allergic eye diseases. These diseases, however, are widely known to be preventable and curable if such production is effectively blocked (Emerging therapeutic targets in asthma and allergy: modulation of IgE. Emerging Therapeutic Targets (1999) 3: 229-240 ; Anti-IgE as novel therapy for the treatment of asthma. Curr. Opin. Plum. Med. (1999) 5: 76-80 ; Treatment of allergic asthma with monoclonal anti-IgE antibody. N. Eng. J. Med. (1999) 341: 1966-1973 ; Anti-IgE antibody therapy for asthma. N. Eng. J. Med.(1999) 341: 2006-2008).

From the foregoing, IgE is considered to be a substance which takes part at the stage of very beginning upon onset of an allergic disease. With the aim of developing antiallergic agents, some low molecular compounds with IgE antibody production inhibiting activity have been found and reported to date [WO98/04058, WO98/07702, WO98/16497, JP10-324631A, WO99/19291, WO99/35140, WO99/38829, WO99/42446, JP11-269192A, WO00/05198, "Yakuri to Chiryo (Basic Pharmacology & Therapeutic)" 22(3), 1369 (1994), JP1-106818A, JP7-17506B, JP8-92216A, JP8-109177A, WO96/11682, JP59-167564A]. These compounds, however, involve problems such as low water solubility, and it is the current circumstance that the aim has not been fully achieved.

An object of the present invention, therefore, is to provide a compound having excellent IgE antibody production inhibiting activity and also a medicine comprising the compound as an active ingredient.

### Disclosure of the Invention

Under such circumstances, the present inventors have proceeded with extensive research. As a result, it has been found that compounds represented by the below-described formula (1) have excellent IgE antibody production inhibiting activity and also good water solubility and hence, are useful as medicines for the prevention or treatment of allergic immune diseases, leading to the completion of the present invention.

Accordingly, the present invention provides a bis(5-aryl-2-pyridyl) derivative represented by the following formula (1) or a salt thereof: 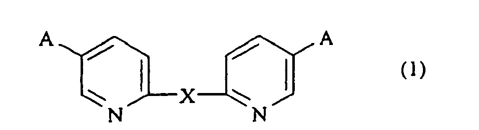 wherein A represents a substituted or unsubstituted aromatic hydrocarbon group or a substituted or unsubstituted aromatic heterocyclic group, and X represents a group selected from the following formulas (2) to (5): 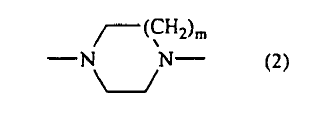 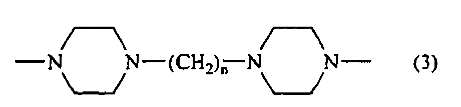 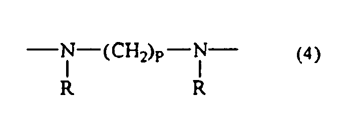 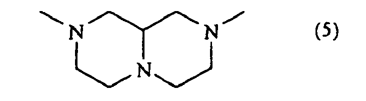 wherein in the formula (2), m stands for an integer of 1 or 2; in the formula (3), n stands for an integer of 1 to 6; in the formula (4), R represents a hydrogen atom or a lower alkyl group and p stands for an integer of 1 to 6.

The present invention also provides a medicine comprising as an active ingredient the bis(5-aryl-2-pyridyl) derivative or the salt thereof.

The present invention also provide use of the bis(5-aryl-2-pyridyl) derivative or the salt for the manufacture of a medicine.

The present invention also provides a medicinal composition comprising the bis (5-aryl-2-pyridyl) derivative or the salt thereof and a pharmacologically acceptable carrier.

The present invention also provides a treatment method for an allergic immune disease, which comprises administering the bis(5-aryl-2-pyridyl) derivative or the salt thereof.

### Best Modes for Carrying Out the Invention

The lower alkyl moieties in "lower alkyl groups", "halogeno(lower alkyl) groups", "hydroxy(lower alkyl) groups", "lower alkoxy(lower alkyl) groups", "lower alkoxy groups", "(lower alkyl)thio groups", "(lower alkyl)amino groups", "(lower alkyl)sulfonylamino groups", "(lower alkoxy)carbonyl groups" and "lower alkanoyl groups" as used herein are linear, branched or cyclic alkyl groups having 1 to 6 carbon atoms. Their examples can include methyl, ethyl, n-propyl, isopropyl, n-butyl, isobutyl, sec-butyl, tert-butyl, pentyl, hexyl, cyclopropyl, and cyclohexyl. On the other hand, illustrative of "halogen atoms" are fluorine atom, chlorine atom, bromine atom, and iodine atom.

In the formula (1), the aromatic hydrocarbon group represented by A may preferably be one having 6 to 14 carbon atoms, with a phenyl or naphthyl group being more preferred and a phenyl group being particularly preferred. Preferred Examples of the aromatic heterocyclic group can include 5- to 10-membered heterocyclic groups each of which contains one or two nitrogen, oxygen or sulfur atoms, with pyridyl, thienyl, furyl, benzofuryl and benzothienyl groups being more preferred.

These groups may contain 1 to 3 substituents. Examples of such substituents can include lower alkyl groups, halogeno(lower alkyl) groups, hydroxy(lower alkyl) groups, lower alkoxy(lower alkyl) groups, lower alkoxy groups, halogen atoms, hydroxy group, cyano group, (lower alkyl)thio groups, amino group, mono- or di-(lower alkyl)amino groups, (lower alkyl)sufonylamino groups, formyl group, carboxyl group, (lower alkoxy)carbonyl groups, lower alkanoyl groups, pyrrolidinyl group, and alkylenedioxy groups.

Preferred specific examples of these substituents can include methyl, t-butyl, trifluoromethyl, hydroxymethyl, methoxymethyl, methoxy, ethoxy, n-propoxy, isopropoxy, F, Cl, hydroxy, cyano, methylthio, amino, dimethylamino, methane-sulfonylamino, pyrrolidinyl, formyl, carboxy, methoxycarbonyl, ethoxycarbonyl, acetyl, and methylenedioxy.

Among the groups represented by X, preferred are the group of the formula (3) in which n ranges from 2 to 4 and the group of the formula (4) in which p ranges form 2 to 4.

Preferred specific examples of the bis(5-aryl-2-pyridyl) derivative (1) according to the present invention can include
1,4-bis[5-(3,4,5-trimethoxyphenyl)-2-pyridyl]hexahydro-1,4-diazepine dimethanesulfonate,
1,4-bis[5-(4-amino-3,5-dimethoxyphenyl)-2-pyridyl]hexahydro-1,4-diazepine tetrahydrochloride,
1,4-bis[5-(4-dimethylamino-3,5-dimethoxyphenyl)-2-pyridyl]hexahydro-1,4-diazepine,
1,3-bis[4-[5-(3,4,5-trimethoxyphenyl)-2-pyridyl]-1-piperazinyl]propane, and
N,N'-bis[5-(3,4,5-trimethoxy-phenyl)-2-pyridyl]-N,N'-dimethylethylenediamine dimethane-sulfonate.

The compound (1) according to the present invention can be produced, for example, in accordance with the following reaction steps, although no particular limitation is imposed on its production process. 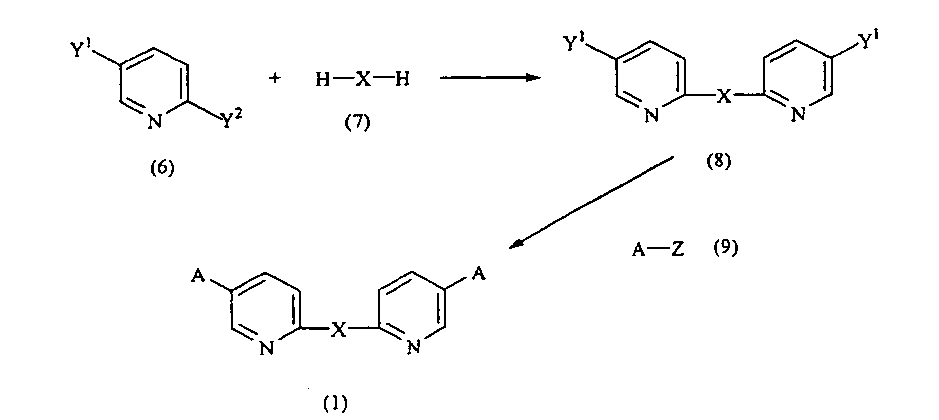 wherein A and X have the same meanings as defined above, Y¹ and Y² represent a halogen atom or -OSO₂(C_{q}F_{2q+1}) in which q stands for an integer of 0 to 4, and Z represents dihydroxyboron, di (lower alkoxy)boron, di(lower alkyl)boron, dihalo(lower alkyl)silicon, halogenated zinc, tri(lower alkyl)tin, halogenated magnesium or the like.

Described specifically, the compound (1) according to the present invention can be produced by reacting the compound (6) with the compound (7) in a solventless manner or in a solvent optionally in the presence of a base to obtain a compound (8) and then reacting the compound (8) with the compound (9).

Examples of the solvent employed in the reaction (condensation reaction) between the compound (6) and the compound (7) can include toluene, tetrahydrofuran, dioxane and dimethylformamide, whereas examples of the base usable in the reaction can include potassium carbonate and sodium hydride. The reaction can be conducted preferably at room temperature to 200°C for 0.5 to 100 hours, notably at 80 to 120°C for 2 to 15 hours.

To obtain the compound (1) according to the present invention, the reaction (cross-coupling reaction) between the compound (8) and the compound (9) can be conducted by adding the compound (9) and a catalyst to a solution or suspension of the compound (8) and allowing the compound (8) to react with the compound (9) optionally in the presence of a ligand and a base [Metal-catalyzed Cross-coupling Reactions; Diederich, F., Stang, P.J., Eds.; Wiley-VHC: Weinheim (1998). Stanforth, S.P. Tetrahedron, 54, 263-303 (1998)].

Illustrative of a solvent usable in the above reaction are benzene, toluene, xylene, diethyl ether, tetrahydrofuran, dimethoxyethane, dioxane, acetonitrile, dimethylformamide, N-methylpiperidone, methanol, ethanol, and water. Examples of the catalyst can include
tetrakis(triphenylphosphine)-palladium(0),
tris(bisbenzylideneacetone)dipalladium(0), palladium acetate(II), palladium chloride(II),
dichloro-bis(triphenylphosphine)palladium(II),
dichloro[1,2-bis(diphenylphosphine)ethane]palladium(II),
dichloro[1,4-bis(diphenylphosphino)butane]palladium(II),
dichloro[1,1-bis(diphenylphosphino)ferrocene]palladium(II),
tetrakis-triphenylphosphine nickel(0) and
bis(acetylacetonato)nickel (II).

Examples of the ligand, on the other hand, can include tri(t-butyl)phosphine, triphenylphosphine,
tri(o-tolyl)-phosphine, tri(2-furyl)phosphine,
2,2'-bis(diphenyl-phosphino)-1,1'-binaphthyl,
1,2-bis(diphenylphosphino)-ethane,
1,3-bis(diphenylphosphino)propane,
1,4-bis(diphenylphosphino)butane, and
1,1'-bis(diphenyl-phosphino)ferrocene. Examples of the base can include sodium acetate, potassium acetate, sodium carbonate, sodium hydrogencarbonate, potassium phosphate, sodium hydroxide, potassium hydroxide, barium hydroxide, sodium methoxide, sodium ethoxide, cesium fluoride, fluorinated tributyl-ammonium, and triethylamine.

As reaction conditions, the reaction may be conducted at room temperature to 150°C for 0.5 to 100 hours. It is, however, preferred to follow the report by Suzuki et al. [Miyaura, N.; Suzuki, A. Chem. Rev., 95, 2457-2483 (1995)], that is, to use a compound (9) in which Z is dihydroxyboron and to apply conditions of tetrakis(triphenylphosphine)palladium(0)/ potassium carbonate (or sodium carbonate)/water-methanol(or ethanol)-toluene/60 to 100°C/0.5 to 3 hours; or to use a compound (9) in which Z is di (lower alkoxy)boron and to apply conditions of dichloro[1,1]-bis(diphenylphosphino)ferrocene]palladium (II)/1,1'-bis(diphenylphosphino)ferrocene/sodium carbonate/water-dimethylformamide/60 to 100°C/0.5 to 3 hours.

The intermediate and target compound obtained in the above reactions, respectively, can be isolated and purified by subjecting them to purification procedures commonly employed in organic synthesis chemistry, for example, filtration, extraction, washing, drying, concentration, recrystallization, one or more of various chromatographic treatments, and the like. Further, the intermediate can be provided for use in the next reaction without specifically purifying it.

In addition, they may also be obtained in the form of solvates with reaction solvents, recrystallization solvents or the like, especially as hydrates. Further, the compound (1) according to the present invention may include various isomers depending on the kinds and combination of the substituents. It is to be noted that the present invention encompasses all of such isomers.

The compound (1) obtained as described above can be converted into an acid addition salt or a basic salt by a method known *per se* in the art. No particular limitation is imposed on such salts insofar as they are pharmacologically acceptable salts. When the compound (1) is a basic compound, examples of its pharmacologically acceptable salt can include mineral acid salts such as the hydrochloride, sulfate and nitrate; and organic acid salts such as the methanesulfonate, acetate, oxalate and citrate. When the compound (1) is an acidic compound, on the other hand, examples of its pharmacologically acceptable salt can include alkali metal salts such as the sodium and potassium salts; alkaline earth metal salts such as the calcium and magnesium salts; and organic base salts such as the pyridine, picoline and triethylamine salts.

As the bis (5-aryl-2-pyridyl) derivative (1) according to the present invention has excellent IgE antibody production inhibiting activity and also, IL-4 production inhibiting activity and IL-5 production inhibiting activity as will be demonstrated in tests to be described subsequently herein, it is useful as medicines for the prevention or treatment of various allergic diseases - for example, asthma, atopic dermatitis, allergic rhinilis, inflammatory colitis, contact skin diseases and allergic eye diseases - and also as an IgE antibody production inhibitor.

The medicine according to the present invention comprises as an active ingredient the bis(5-aryl-2-pyridyl) derivative or the salt thereof. By adding pharmacologically acceptable, inorganic or organic carriers, the bis(5-aryl-2-pyridyl) derivative or the salt thereof can be formulated into medicinal compositions, for example, various oral preparations or parenteral preparations such as solid, semi-solid or liquid preparations by methods known *per se* in the art.

Illustrative of preparations for oral administration are tablets, pills, granules, soft or hard capsules, triturates, subtilized granules, powders, emulsions, syrups, pellets, and elixirs. On the other hand, illustrative of preparations for parenteral administration are injections, drips; infusions, ointments, lotions, tonics, sprays, suspensions, medicinal oils, emulsions, suppositories, and instillations.

To formulate such preparations, methods known *per se* in the art can be followed. Active ingredients according to the present invention can be used in combination with pharma-cologically acceptable surfactants, excipients, coloring matters, flavoring agents, preservatives, stabilizers, buffering agents, suspending agents, isotonicities and the like as needed.

The dosage of the medicine according to the present invention varies inter alia depending on the medicine, the target disease to be treated or prevented, the administration method, the treatment period, and the age, sex and weight of the patient. Nonetheless, it is preferred to administer the medicine at a daily dosage of from 0.01 to 1,000 mg/kg weight in terms of the compound represented by the formula (1). This dosage can be administered at once or in several portions, for example, 2 to 6 portions in a day.

### Examples

The present invention will next be described further based on Examples. It should, however, be borne in mind that the present invention is not limited to or by the following Examples.

### Referential Example 1

### 1,4-Bis(5-bromo-2-pyridyl)hexahydro-1,4-diazepine

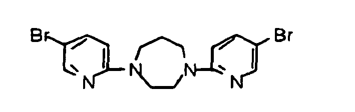

To a solution of hexahydro-1,4-diazepine (2.84 g, 28.4 mmol) in dimethylformamide (14 mL), 2,5-dibromopyridine (16.23 g, 68.5 mmol) and potassium carbonate (8.00 g, 57.9 mmol), said potassium carbonate having had been ground into powder in a mortar, were added, and the resultant mixture was stirred at 120°C for 15 hours. Water was added to the reaction mixture, followed by extraction with chloroform. The organic layer was dried over anhydrous sodium sulfate and then concentrated under reduced pressure. The residue was purified by chromatography on a silica gel column, and crude crystals of the title compound were obtained (7.67 g, yield: 66%). The crude crystals were recrystallized fromchloroform-hexane, whereby colorless fine needles (melting point: 178.0-181.0°C) were obtained.

### Referential Example 2

### 3,5-Dimethoxyphenylboric acid

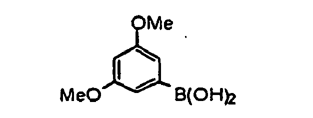

To anhydrous tetrahydrofuran (9. 0 mL) held under stirring in a dry ice-methanol bath, a 1.57 M hexane solution (3.9 mL, 5.9 mmol) of n-butyllithium was added dropwise under an argon gas atmosphere, followed by the gradual dropwise addition of a solution of 3,5-dimethoxyiodobenzene (713.0 mg, 2.70 mmol) in anhydrous tetrahydrofuran (5.0 mL). After the resulting mixture was stirred for 20 minutes in the dry ice-methanol bath, triisopropyl borate (0.75 mL, 3.2 mmol) was added and then, the mixture was stirred for 20 minutes. The reaction mixture was stirred at room temperature for 1 hour and then concentrated under reduced pressure. To the residue, a 1.0 M aqueous solution of sodium hydroxide (7.0 mL) was added. The resulting aqueous solution was washed with chloroform, to which concentrated hydrochloric acid was added to acidify the same, followed by the extraction with chloroform. The organic layer was washed with a saturated aqueous solution of sodium chloride (hereinafter called "brine"), dried over anhydrous sodium sulfate, and then concentrated under reduced pressure. The residue was recrystallized from chloroform-hexane, whereby the title compound was obtained as colorless crystalline powder (melting point: 159.0-161.0°C) (367.0 mg, yield: 91%).

### Referential Example 3

### 3,5-Diisopropoxyphenyl trifluoromethanesulfonate

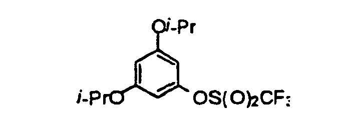

To a solution of 3,5-diisopropoxyphenol [J. Chem. Soc. Perkin Trans 1, 17, 2939-2942 (1998)] (170.0 mg, 0.810 mmol) in methylene chloride (4.5 mL), N,N-diisopropylethylamine (195.0 mg, 1.50 mmol) was added. The resulting solution was cooled in a dry ice-methanol bath, followed by the gradual dropwise addition of trifluoromethanesulfonic acid anhydride (367.0 mg, 1.30 mmol) over approx. 10 minutes. Subsequent to stirring for 3 hours, brine was added to the reaction mixture, and the mixture was extracted with chloroform. The organic layer was dried over anhydrous sodium sulfate and then concentrated under reduced pressure. The residue was purified by chromatography on a silica gel column, whereby the title compound was obtained as a pale yellow oil (261.0 mg, yield: 94%).

### Referential Example 4

### 3,5-Bis(methoxymethyl)phenyl trifluoromethanesulfonate

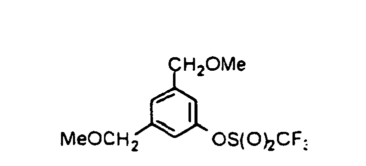

To a solution of dimethyl 5-hydroxyphthalate [J. Org. Chem., 65, 5360-5370 (2000)] (1.25 g, 5.95 mmol) in anhydrous dimethylformamide (10 mL), said solution having had been stirred under ice cooling, imidazole (0.83 g, 12.2 mmol) and t-butylchlorodipyhenylsilane (2.2 mL, 8.5 mmol) were added. After the resultant mixture was stirred under ice cooling for 15 minutes and then at room temperature for 2 hours, water was added. The mixture was extracted with benzene-hexane (1:1). The organic layer was dried over anhydrous sodium sulfate and then concentrated under reduced pressure, whereby an oil (3.40 g) containing dimethyl 5-(t-butyldiphenylsiloxy)isophthalate was obtained.

Under ice cooling, lithium aluminum hydride (1.00 g, 26.4 mmol) was added to a solution of the oil (3.22 g), which had been obtained by the above-described procedures, in anhydrous tetrahydrofuran (35 mL), and the resultant mixture was stirred for 20 minutes. Methanol (7.0 mL, 170 mmol) was added to the reaction mixture, and the ice bath was removed. Water (7.0mL), diethyl ether (150 mL) and anhydrous magnesium sulfate (30 g) were added. After the mixture was stirred at room temperature for 3 hours, insoluble matter was filtered off by using Celite. The residue was washed with chloroform. The filtrate and the washing were combined, followed by concentration under reduced pressure. The residue was recrystallized from diethyl ether-hexane, whereby
1-t-butylphenylsiloxy-3,5-bis(hydroxymethyl)benzene was obtained as colorless needles (melting point:
132.0-133.0°C) (1.76 g, yield: 75% based on the dimethyl 5-hyroxyisophthalate).

Under ice cooling, methyl iodide (0.80 mL, 13 mmol) and a 50% dispersion of sodium hydride (156.5 mg, 3.26 mmol) were added to a solution of
1-t-butyldiphyenylsiloxy-3,5-bis(hydroxymethyl)benzene (501.3 mg, 1.28 mmol) in dimethylformamide (5.0 mL), and the resulting mixture was stirred for 1 hour. Water was added to the reaction mixture, and the thus-obtained mixture was extracted with benzene-hexane (1:1). The organic layer was dried over anhydrous sodium sulfate and then concentrated under reduced pressure, whereby an oil (0.60 g) containing
1-t-butyldiphenylsiloxy-3,5-di(methoxymethyl)benzene was obtained.

Under ice cooling, a 1.0 M tetrahydrofuran solution (2.0 mL) of tetrabutylammonium fluoride (2.0 mmol) was added to a solution of the oil (0.60 g), which had been obtained by the above-described procedures, in tetrahydrofuran (8.0 mL), and the resulting mixture was stirred for 15 minutes. An approx. 20% aqueous solution of ammonium chloride was added, and the mixture so obtained was extracted with diethyl ether. The organic layer was dried over anhydrous sodium sulfate and then concentrated under reduced pressure. The residue was purified by chromatography on a silica gel column, whereby
3,5-bis(methoxymethyl)phenol was obtained as a colorless oil [219.8 mg, yield: 94% based on the 1-t-butyldiphenylsiloxy-3,5-bis(hydroxymethyl)benzene].

By similar procedures as in Referential Example 3, the title compound was obtained as a colorless oil (343.9 mg, yield: 93%) from 3,5-bis(methoxymethyl)phenol (215.4 mg, 1.18 mmol).

### Referential Example 5

### 3,4,5-Triethoxyphenylboric acid

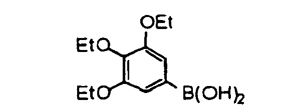

A solution of 3,4,5-triethoxybenzoic acid (20.0 g, 78.7 mmol) in acetic acid (68 mL) was stirred at 15°C, followed by the addition of concentrated nitric acid (d 1.38) (34 mL, 750 mmol). After stirred at room temperature for 1 hour, the reaction mixture was poured into ice water and then extracted with ethyl acetate. The organic layer was washed with a saturated aqueous solution of sodium-hydrogencarbonate and brine, dried over anhydrous sodium sulfate and then concentrated under reduced pressure. The residue was purified by chromatography on a silica gel column, whereby 3,4,5-triethoxy-1-nitrobenzene was obtained as a colorless oil (12.9 g, yield: 64%).

To a solution of 3,4,5-triethoxy-1-nitrobenzene (12.9 g, 50.5 mmol) in methanol (445 mL), 10% palladium on charcoal (6.7 g) and ammonium formate (16.2 g, 253 mmol) were added, and the resulting mixture was stirred at 70°C for 1 hour. Insoluble matter was filtered off by using Celite, and the filtrate was concentrated under reduced pressure. A solution of the residue in chloroform was washed with water and saturated brine, dried over anhydrous sodium sulfate and then concentrated under reduced pressure, whereby an oil (10.5 g) containing
3,4,5-triethoxyaniline was obtained.

The oil (9.5 g, approx. 42.2 mmol) obtained by the above procedures was suspended in water (200 mL). Under ice cooling, concentrated sulfuric acid (7.3 mL, 88 mmol) was added to the suspension to provide a homogeneous solution. Under ice cooling, a solution of sodium nitrite (3.10 g, 44.0 mmol) in water (10 mL) was gradually added dropwise to the solution over approx. 10 minutes, and the solution was stirred for 15 minutes, followed by the addition of a solution of potassium iodide (7.70 g, 46.0 mmol) in water (10 mL). The reaction mixture was stirred at room temperature for 1 hour and at 50°C for 15 minutes, and was then extracted with ethyl acetate. The organic layer was washed with brine, dried over anhydrous sodium sulfate, and then concentrated under reduced pressure. The residue was purified by chromatography on a silica gel column, whereby
3,4,5-triethoxy-1-iodobenzene was obtained as pale yellow needles (melting point: 44.0-46.0°C) (11.60 g, yield: 75%).

By similar procedures as in Referential Example 2, crude crystals were obtained from 3,4,5-triethoxy-1-iodobenzene (5.00 g, 14.9 mmol). The crude crystals were recrystallized from chloroform-hexane, whereby the title compound was obtained as colorless needles (melting point: 167.0-168.0°C) (1.80 g, yield: 48%).

### Referential Example 6

### 3,5-Diisopropoxy-4-methoxyphenylboric acid

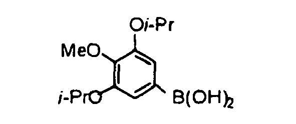

To a solution of methyl 3, 5-dihydroxy-4-methoxybenzoate [Ann. Chem., 544, 62-71 (1940)] (4.40 g, 22.2 mmol) in dimethylformamide (44.0 mL), potassium carbonate (10.20 g, 73.8 mmol) and isopropyl iodide (19.66 g, 115 mmol) were added. After stirred at 70°C for 3 hours, water was added to the reaction mixture. The resulting mixture was extracted with ethyl ether. The organic layer was dried over anhydrous sodium sulfate and then concentrated under reduced pressure. The residue was purified by chromatography on a silica gel column, whereby methyl 3,5-diisopropoxy-4-methoxybenzoate was obtained as a pale yellow oil (4.16 g, yield: 66%).

To a solution of methyl 3,5-diisopropoxy-4-methoxy-benzoate (4.16 g, 14.7 mmol) in methanol (20 mL), a 5.0 M aqueous solution of sodium hydroxide (20.0 mL, 100 mmol) was added. After stirred at 80°C for 3 hours, the reaction mixture as concentrated under reduced pressure to remove methanol. Concentrated hydrochloric acid was added to the residue to acidify the same, and the resulting mixture was extracted with chloroform. The organic layer was washed with brine, dried over anhydrous sodium sulfate and then concentrated under reduced pressure, whereby an oil (4.00 g) containing 3,5-diisopropoxy-4-methoxybenzoic acid was obtained.

The following synthesis procedures were conducted in accordance with the procedures of Referential Example 5. From the oil (3.70 g) obtained by the above-described procedures, 3,5-diisopropoxy-4-methoxy-1-nitrobenzene (1.70 g) was obtained as a pale yellow oil (yield: 46% based on the methyl 3,5-diisopropoxy-4-methoxybenzoate). From 3,5-diisopropoxy-4-methoxy-1-nitrobenzene (1.70g, 6.30mmol), 1-iodo-3,5-diisopropoxy-4-methoxybenzene was obtained as a pale yellow oil (1.45 g, yield: 66%). From 1-iodo-3,5-diisopropoxy-4-methoxybenzene (700.0mg, 2.17 mmol), the title compound was obtained as colorless crystalline powder (melting point: 159.0-161.0°C)(367.0 mg, yield: 70%).

### Referential Example 7

### 3-Methoxy-4,5-methylenedioxyphenylboric acid

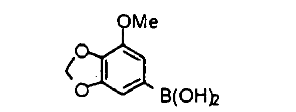

To a solution of 3-hydroxy-4,5-methylenedioxybenzoic acid [Liebigs Ann. Chem., 361-364 (1994)], (3.90 g, 20.0 mmol) in dimethylformamide (20.0 mL), potassium carbonate (2.80 g, 20.0 mmol) and methyl iodide (4.30 g, 30.0 mmol) were added, and the resultant mixture was stirred at 50°C for 1 hour. Water was added to the reaction mixture, and the thus-obtained mixture was extracted with diethyl ether. The organic layer was dried over anhydrous sodium sulfate and then concentrated under reduced pressure. The residue was purified by chromatography on a silica gel column, whereby methyl 3-methoxy-4,5-methylenedioxybenzoate was obtained as a colorless viscous oil (4.30 g, yield: 98%).

Synthesis of the title compound from methyl 3-methoxy-4,5-methylenedioxybenzoate was conducted by applying the synthesis procedures from methyl 3,5-diisopropoxy-4-methoxybenzoate to 3,5-diisopropoxy-4-methoxyphenylboric acid in Referential Example 6. From methyl 3-methoxy-4,5-methylenedioxybenzoate (4.30 g, 20.5 mmol), 3-methoxy-4,5-methylenedioxybenzoic acid (3.00 g) was obtained as colorless prisms (melting point: 215.0-216.0°C) (yield: 71%). From 3-methoxy-4,5-methylenedioxy-benzoic acid (2.50 g, 12.7 mmol), 3-methoxy-4,5-methylenedioxy-1-nitrobenzene was obtained as pale yellow amorphous powder (1.26 g, yield: 50%). From 3-methoxy-4,5-methylenedioxy-1-nitrobenzene (1.26 g, 16.4 mmol), 1-iodo-3-methoxy-4,5-methylenedioxybenzene was obtained as pale yellow crystals (melting point: 71.0-72.0°C) (1.08 g, yield: 61%). From 1-iodo-3-methoxy-4,5-methylene-dioxybenzene (750.0 mg, 2.70 mmol), the title compound was obtained as colorless crystalline powder (melting point: 279.0-281.0°C) (347.0 mg, yield: 66%).

### Referential Example 8

### 4-Benzyloxy-3,5-dimethoxyphenylboric acid

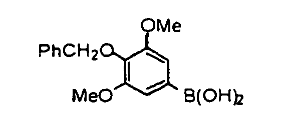

To a solution of methyl syringate (6.00 g, 28.3 mmol) in dimethylformamide (61.0 mL), potassium carbonate (2.80 g, 20.3 mmol) and benzyl bromide (5.80 g, 33.9 mmol) were added, and the resulting mixture was stirred at 50°C for 2 hours. After the mixture was allowed to cool down, water was added, followed by the extraction of the resulting mixture with diethyl ether. The organic layer was washed with brine, dried over anhydrous sodium sulfate and then concentrated under reduced pressure, whereby an oil (7.90 g) containing methyl 4-benzyloxy-3,5-dimethoxybenzoate was obtained.

To a solution of the oil (7.90 g), which had been obtained by the above-described procedures, in methanol (40 mL), a 5.0 M aqueous solution of sodium hydroxide (40 mL, 200 mmol) was added. After stirred at 80°C for 30 minutes, the reaction mixture was concentrated under reduced pressure to remove methanol. Concentrated hydrochloric acid was added to the residue to acidify the same, and the resulting mixture was extracted with chloroform. The organic layer was washed with brine, dried over anhydrous sodium sulfate and then concentrated under reduced pressure, whereby an oil (6.40 g) containing 4-benzyloxy-3,5-dimethoxybenzoic acid was obtained.

To a solution of the oil (6.40 g), which had been obtained by the above-described procedures, in t-butyl alcohol (96 mL), triethylamine (2.43 g, 23. 6 mmol) and diphenylphosphoryl azide (6.40 g, 23.6 mmol) were added. After stirred at 100°C for 2 hours, the reaction mixture was concentrated under reduced pressure. A solution of the residue in ethyl acetate was washed with a saturated aqueous solution of sodium hydrogencarbonate and brine, dried over anhydrous sodium sulfate and then concentrated under reduced pressure. The residue was purified by chromatography on a silica gel column, whereby 4-benzyloxy-N-t-butoxycarbonyl-3,5-dimethoxyaniline was obtained as a colorless oil (4.56 g, yield: 44% based on the methyl syringate).

To a solution of 4-benzyloxy-N-t-butoxycarbonyl-3,5-dimethoxyaniline (590.0 mg, 1.64 mmol) in methanol (2.0 mL), a 4.0 M ethyl acetate solution of hydrogen chloride (2.0 mL, 8.0 mmol) was added, and the resulting mixture was stirred at 50°C for 1 hour. The reaction mixture was concentrated under reduced pressure, a 8.0 M aqueous solution of sodium hydroxide (1.5 mL, 12 mmol) was added to the residue, and the thus-obtained mixture was extracted with chloroform. The organic layer was washed with brine, dried over anhydrous sodium sulfate and then concentrated under reduced pressure, whereby an oil (426.0 mg) containing 4-benzyloxy-3,5-dimethoxyaniline was obtained.

Synthesis of the title compound from the oil obtained by the above-described procedures was conducted by applying the synthesis procedures from the oil, which contained 3,4,5-triethoxyaniline, into 3,4,5-triethoxyphenylboric acid in Referential Example 5. From the oil (426.0 mg, approx. 1.64 mmol) obtained by the above-described procedures, 4-benzyloxy-1-iodo-3,5-dimethoxybenzene was obtained as a colorless oil (447.0 mg, yield: 73% based on the 4-benzyloxy-N-t-butoxycarbonyl-3,5-dimethoxyaniline). From 4-benzyloxy-1-iodo-3,5-dimethoxybenzene (447.0 mg, 1.21 mmol), the title compound was obtained as colorless crystalline powder (melting point: 180.0-183.0°C) (87.0 mg, yield: 25%).

### Referential Example 9

### 3,5-Dimethoxy-4-methylthiophenylboric acid

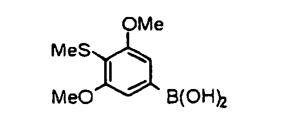

To a solution of 4-bromo-3,5-dimethoxybenzoic acid [Acta Chem. Scand., 2, 34-41, (1948)] (22.6 g, 86. 6 mmol) in methylene chloride (400 mL), dimethylformamide (1.5 mL, 19.4 mmol) was added. Under ice cooling, oxalyl chloride (13.0 g, 102.0 mmol) was gradually added to the solution, and the resulting mixture was stirred at room temperature for 1 hour and 30 minutes. The reaction mixture was concentrated under reduced pressure, whereby crude crystals of 4-bromo-3,5-dimethoxybenzoyl chloride were obtained. To an ice-cooled solution of 2-amino-2-methylpropanol (8.40 g, 94.2 mmol) in methylene chloride (100 mL), N,N-diisopropylethylamine (16.6 mL, 95.3 mmol) and a solution of the crude 4-bromo-3,5-dimethoxytbenzoyl chloride in methylene chloride (200 mL) were added. After the thus-obtained mixture was stirred at room temperature for 10 minutes, the mixture was washed successively with water, 8.0 M hydrochloric acid, a saturated aqueous solution of sodium hydrogencarbonate and brine, dried over anhydrous sodium sulfate, and then concentrated under reduced pressure. Thionyl chloride (47.5 mL, 651.0 mmol) was added to the residue, and the resulting mixture was stirred at room temperature for 10 minutes. To the reaction mixture, ice water and an 2.5 M aqueous solution of sodium hydroxide (600 mL, 1500 mmol) were added, and the thus-obtained mixture was extracted with diethyl ether. The organic layer was washed with brine, dried over anhydrous sodium sulfate, and then concentrated under reduced pressure. The residue was recrystallized form diethyl ether-hexane, whereby 2-(4-bromo-3,5-dimethoxyphenyl)-4,4-dimethyl-2-oxazoline was obtained as colorless fine needles (melting point: 172.5 to 179.5°C) (20.9 g, yield: 77%).

Under an argon gas atmosphere, N,N,N',N'-tetramethyl-ethylenediamine (30.0mL, 0.199mol) and a 1.59 M hexane solution of n-butyl lithium (134.0 mL, 0.213 mol) were added dropwise to a solution of 2-(4-bromo-3,5-dimethoxyphenyl)-4,4-dimethyl-2-oxazoline (51.43 g, 0.164 mmol) in anhydrous tetrahydrofuran (1000 mL) which was held under stirring in a dry ice-methanol bath. After stirred for 10 minutes, dimethyl disulfide (18. 0 mL, 0.200 mmol) was added. After stirred further for 1 hour, water was added to the reaction mixture, the organic solvent was removed by concentration under reduced pressure, and the residue was extracted with diethyl ether. The organic layer was washed with brine, dried over anhydrous sodium sulfate and then concentrated under reduced pressure. The residue was recrystallized form diethyl ether-hexane, whereby 2-(4-methylthio-3,5-dimthoxyphenyl)-4,4-dimethyl-2-oxazolin e was obtained as colorless fine needles (melting point: 82.5 to 84.5°C)(39.80 g, yield: 76%).

A solution of 2-(4-methylthio-3,5-dimethoxyphenyl)-4,4-dimethyl-2-oxazoline (34.70 g, 0.123 mmol) in 3.0 M hydrochloric acid (450 mL) was stirred at 100°C for 3 hours, and the reaction mixture was extracted with methanol-chloroform (1:10). The organic layer was washed with brine, dried over anhydrous sodium sulfate, and then concentrated under reduced pressure. To a solution of the residue in methanol (50 mL), a 2.5 M aqueous solution of sodiumhydroxide (100mL, 0.25mol) was added, and the resulting mixture was stirred at 100°C for 1 hour. Under ice cooling, concentrated hydrochloric acid was added to the reaction mixture to acidify the same, and the mixture was extracted with methanol-chloroform (1:10). The organic layer was washed with brine, dried over anhydrous sodium sulfate and then concentrated under reduced pressure, whereby an oil (30.72 g) containing 3,5-dimethoxy-4-methylthiobenzoic acid was obtained.

Synthesis of the title compound from the oil obtained by the above-described procedures was conducted by applying the synthesis procedures from the oil, which contained 4-benzyloxy-3,5-dimethoxybenzoic acid, into 4-benzyloxy-3,5-dimethoxyphenylboric acid in Referential Example 8. From the oil (30.72g) obtained by the above-described procedures, N-t-butoxycarbonyl-3,5-dimethoxy-4-methylthioaniline was obtained as colorless fine crystals (melting point: 123.5-125.5-C) [26.33 g, yield: 71% based on 2-(4-methylthio-3,5-dimethoxyphenyl)-4,4-dimethyl-2-oxazoline]. From N-t-butoxycarbonyl-3,5-dimethoxy-4-methylthioaniline (12.74 g, 42.6 mmol), 1-iodo-3,5-dimethoxy-4-methylthiobenzene was obtained as brown crystalline powder (melting point: 103.0-104.0°C) (8.90 g, yield: 67%). From 1-iodo-3,5-dimethoxy-4-methylthiobenzene (6.90 g, 22.2 mmol), the title compound was obtained as colorless crystalline powder (melting point: 262.0-265.0°C)(4.10 g, yield: 82%).

### Referential Example 10

### 4-Chloro-3,5-dimethoxyphenylboric acid

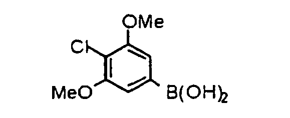

Under ice cooling, a solution of sodium nitrite (97.0 mg, 1.40 mmol) in water (2.0 mL) was added dropwise to a suspension of 4-bromo-2,6-dimethoxyaniline [Z. Naturforsch., B24(5), 524-527 (1969)] (232.0 mg, 1.00 mmol) in 6.0 M hydrochloric acid (2.5 mL). After the resulting mixture was stirred under ice cooling for 30 minutes, a solution of cupric chloride (495.0 mg, 5.00 mmol) in concentrated hydrochloric acid (2.0 mL) was added. The reaction mixture was stirred at room temperature for 30 minutes and then at 100°C for 2 hours, and was then extracted with ethyl acetate. The organic layer was washed with a saturated aqueous solution of sodium hydrogencarbonate and water, dried over anhydrous sodium sulfate, and then concentrated under reduced pressure. The residue was purified by chromatography on a silica gel column, whereby 1-bromo-4-chloro-3,5-dimethoxybenzene was obtained as colorless amorphous powder (230.0 mg, yield: 92%).

By similar procedures as in Referential Example 2, crude crystals were obtained from 1-bromo-4-chloro-3,5-dimethoxy-benzene (160.0mg, 0.630mmol). The crude crystals were purified by chromatography on a silica gel column, whereby the title compound was obtained as colorless amorphous powder (90.0 mg, yield: 66%).

### Referential Example 11

### 4-Cyano-3,5-dimethoxyphenylboric acid

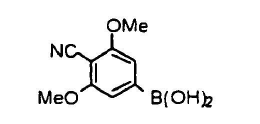

Following the procedures of the synthesis of 3,4,5-triethoxy-1-iodobenzene from the oil containing 3,4,5-triethoxyaniline in Referential Example 5, 1-iodo-3,5-dimethoxy-4-methylbenzene was obtained as pale yellow amorphous powder (7.59 g, yield: 87%) from 3,5-dimethoxy-4-methylanilin [J. Chem. Soc., 497-506 (1963)] (5.23 g, 31.3 mmol).

To a solution of 1-iodo-3,5-dimethoxy-4-methylbenzene (8.05 g, 28.9 mmol) in pyridine (83 mL), potassium permanganate (27.4 g, 173.4 mmol) was added, and the resulting mixture was stirred at room temperature for 30 minutes and then at 50°C for 2 hours. Insoluble matter was filtered off by using Celite, and the residue was washed with a 0.1 M aqueous solution of sodium hydroxide. The filtrate and the washing were combined, followed by extraction with diethyl ether. Concentrated hydrochloric acid was added to the water layer to acidify the same, and the thus-obtained mixture was extracted with chloroform. The organic layer was washed with brine, dried over anhydrous sodium sulfate, and then concentrated under reduced pressure. The residue was suspended in hexane and filtration was then conducted, whereby 4-iodo-2,6-dimethoxybenzoic acid (5.57 g) was obtained as pale brown crystalline powder (melting point: 197.5-204.0°C) (5.57 g, yield: 63%).

Under ice cooling, dimethylformamide (15 mg, 0.21 mmol) and oxalyl chloride (0.90 mL, 9.8 mmol) were added to a solution of 4-iodo-2,6-dimethoxybenzoic acid (2.00 g, 6.50 mmol) in methylene chloride (10 mL). The resulting mixture was stirred for 20 minutes, followed by the addition of concentrated aqueous ammonia (20 mL, 450 mmol). The reaction mixture was stirred at room temperature for 20 minutes and then concentrated under reduced pressure, whereby an oil (2.00 g) containing 4-carbamoyl-1-iodo-3,5-dimethoxybenene was obtained.

To a solution of the oil (2.00 g), which had been obtained by the above-described procedures, in tetrahydrofuran (20 mL), carbon tetrachloride (10.0 mL, 104 mmol) and triphenylphosphine (3.50 g, 13.00mmol) were added. The reactionmixture was stirred at 0°C for 20 hours, and then concentrated under reduced pressure. The residue was purified by chromatography on a silica gel column, whereby 4-cyano-1-iodo-3,5-dimethoxy-benzene was obtained as a colorless oil (1.55 g, yield: 82% based on the 4-iodo-2,6-dimethoxybenzoic acid).

Following the procedures of Referential Example 2, the title compound was obtained as colorless amorphous powder (360.0 mg, yield: 72%) from 4-cyano-1-iodo-3,5-dimethoxy-benzene (700.0 mg, 2.42 mmol).

### Referential Example 12

### 4-(t-Butyldimethylsiloxy)methyl-3,5-dimethoxyphenyl trifluoromethanesulfonate

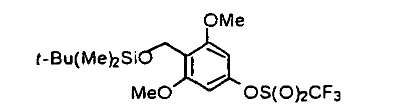

To a solution of 3, 5-dimethoxyphenol (6.73 g, 43.7 mmol) in anhydrous dimethylformamide (50 mL), imidazole (5.95 g, 87.4 mmol) and t-butylchlorodiphenylsilane (15.0 g, 54.6 mmol) were added, followed by stirring at 50°C for 4 hours. Water was added to the reaction mixture, and the resulting mixture was extracted with ethyl acetate-hexane (1:2). The organic layer was washed with water, dried over anhydrous sodium sulfate, and then concentrated under reduced pressure. The residue was purified by chromatography on a silica gel column, whereby 1-t-butyldiphenylsiloxy-3,5-dimethoxybenzene was obtained as colorless crystalline powder (melting point: 95.5-96.5°C) (16.14 g, yield: 94%).

Under a nitrogen gas atmosphere, a 1.60 M hexane solution of n-butyl lithium (31 mL, 49 mmol) was added to a solution of 1-t-butyldiphenylsiloxy-3,5-dimethoxybenzene (14.68 g, 39.0 mmol) in anhydrous diethyl ether (200 mL) which was held under stirring in an ice bath. The reaction mixture was stirred at 35°C for 3 hours and was again cooled in the ice bath, followed by the addition of dimethylformamide (9.21 mL, 119.4 mmol). After stirred at room temperature for 1 hour, a saturated aqueous solution of ammonium chloride was added, and the resulting mixture was extracted with diethyl ether. The organic layer was dried over anhydrous magnesium sulfate, and then concentrated under reduced pressure. The residue was purified by chromatography on a silica gel column, whereby 4-t-butyldiphenylsiloxy-2,6-dimethoxybenzaldehyde was obtained as colorless crystalline powder (melting point: 177.5-120.0°C) (6.77 g, yield: 41%).

Under ice cooling, sodium borohydride (519.0 mg, 13.73 mmol) was added to a solution of 4-t-butyldiphenylsiloxy-2,6-dimethoxybenzaldehyde (3.85 g, 9.15 mmol) in tetrahydrofuran (40 mL), and the resulting mixture was stirred at room temperature for 2 hours and 30 minutes. Water was added to the reaction mixture, and the thus-obtained mixture was extracted with ethyl acetate. The organic layer was washed with brine, dried over anhydrous sodium sulfate and then concentrated under reduced pressure, whereby crude 4-t-butyldiphenylsiloxy-2,6-dimethoxybenzyl alcohol was obtained as colorless crystalline powder (melting point: 127.5-129.0°C) (4.01 g).

To a solution of the crude 4-t-butyldiphenylsiloxy-2,6-dimethoxybenzyl alcohol (4.01 g), which had been obtained by the above-described procedures, in anhydrous dimethyl-formamide (30 mL), imidazole (1.29 g, 18.98 mmol) and t-butylchlorodimethylsilane (1.79 g, 11.86 mmol) were added. After stirred at 50°C for 4 hours, water was added and the resulting mixture was extracted with ethyl acetate-hexane (1:2). The organic layer was washed with water, dried over anhydrous sodium sulfate and then concentrated under reduced pressure, whereby crude 4-(t-butyldimethylsiloxy)methyl-1-t-butyl-diphenylsiloxy-3, 5-dimethoxybenzene was obtained as colorless amorphous powder (5.33 g).

To a solution of the crude 4-(t-butyldimethyl-siloxy)methyl-1-t-butyldiphenylsiloxy-3, 5-dimethoxybenzene (5.33 g), which had been obtained by the above-described procedures, in tetrahydrofuran (60 mL), a 1.0 M tetrahydrofuran solution of tetrabutylammonium fluoride (9.9 mL, 9.9 mmol) was added, After stirred at room temperature for 5 minutes, brine was added and the thus-obtained mixture was extracted with ethyl acetate. The organic layer was dried over magnesium sulfate and then concentrated under reduced pressure. The residue waspurifiedby chromatography on a silica gel column, whereby 4-(t-butyldimethylsiloxy)methyl-3,5-dimethoxyphenol was obtained as colorless amorphous powder (1.93 g, yield: 75% based on the 4-(t-butyldiphenylsiloxy-2,6-dimethoxybenzaldehyde).

Following the procedures procedures of Referential Example 3, the title compound was obtained as colorless amorphous powder from 4-(t-butyldimethylsiloxy)methyl-3,5-dimethoxy-phenol (1.93 g, 6.79 mmol)(1.57 g, yield: 54%).

### Referential Example 13

### 4-Ethoxycarbonyl-1-iodo-3,5-dimethoxybenzene

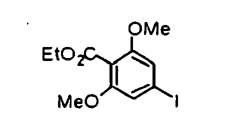

To a solution of 4-iodo-2,6-dimethoxybenzoic acid (603.0 mg, 1.96 mmol), which had been synthesized by the process described in Referential Example 11, in methylene chloride (12 mL), dimethylformamide (0.020 mL, 0.26 mmol) and oxalyl chloride (0.26mL, 3.0 mmol) were added. After stirred at room temperature for 30 minutes, the resulting solution was poured into a solution of triethylamine (0.50 mL, 3.6 mmol) in ethanol (10 mL). The reaction mixture was stirred at room temperature for 30 minutes and then concentrated under reduced pressure. A solution of the residue in diethyl ether was washed successively with water, 0.1 M hydrochloric acid and brine, dried over anhydrous sodium sulfate, and then concentrated under reduced pressure. The residue was purified by chromatography on a silica gel column, whereby the title compound was obtained as a colorless oil (641.0 mg, yield: 97%).

### Referential Example 14

### 4-t-Butoxycarbonyl-3,5-dimethoxyphenylboric acid

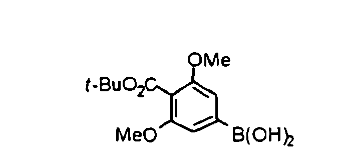

Following the procedures of in Referential Example 13, crude 4-t-butoxycarbonyl-1-iodo-3,5-dimethoxybenzene was obtained as colorless amorphous powder (2.33 g) from 4-iodo-2,6-dimethoxybenzoic acid (2.40 g, 7.79 mmol).

Following the procedures of Referential Example 2, an oil was obtained from the crude 4-t-butoxycarbonyl-1-iodo-3,5-dimethoxybenzene (1.99 g, approx. 5.48 mmol) obtained by the above-described procedures. Diethyl ether-hexane was added to the oil and the resulting precipitate was collected by filtration, whereby the title compound was obtained as colorless crystalline powder (melting point: ≥300.0°C) (1.06 g, yield: 56% based on the 4-iodo-2,6-dimethoxybenzoic acid).

### Referential Example 15

### 4-Acetyl-3,5-dimethoxyphenyl trifluoromethanesulfonate

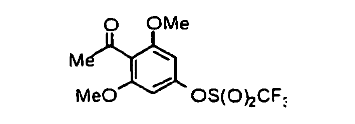

Under a nitrogen gas atmosphere, a solution of the 1-t-butyldiphenylsiloxy-3,5-dimethoxybenene (7.85 g, 22.0 mmol), which had been synthesized by the process described in Referential Example 12, in anhydrous diethyl ether (80 mL) was stirred in an ice bath, followed by the addition of a 1.59 M hexane solution of n-butyl lithium (16.6 mL, 26.6 mmol). After the reaction mixture was stirred at 35°C for 1 hour and 30minutes, it was again stirred in the ice bath, followed by the addition of acetyl chloride (2. 34 mL, 33.0 mmol). The resulting mixture was stirred at room temperature for 30 minutes, followed by the addition of a saturated aqueous solution of ammonium chloride. The mixture so obtained was extracted with diethyl ether. The organic layer was washed with water, dried over anhydrous magnesium sulfate, and then concentrated under reduced pressure. The residue was purifiedby chromatography on a silica gel column, whereby 4-t-butyldiphenylsiloxy-2,6-dimethoxy-acetophenone was obtained as a colorless oil (2.07 g, yield: 22%).

To a solution of 4-t-butyldiphenylsiloxy-2,6-dimethoxyacetophenone (2.07 g, 4.76 mmol) in tetrahydrofuran (42 mL), a 1.0 M tetrahydrofuran solution of tetrabutylammonium fluoride (5.2 mL, 5.2 mmol) was added. The reaction mixture was stirred at room temperature for 5 minute and then concentrated under reduced pressure. A solution of the residue in methylene chloride (63 mL) was stirred in a dry ice-methanol bath. Subsequent to the addition of N,N-diisopropylethylamine (2.1 mL, 12 mmol), trifluoromethanesulfonic anhydride (1.2 mL, 7.1 mmol) was gradually added dropwise. The reaction mixture was stirred for 30 minutes in the dry ice-methanol bath, washed with 1.0 M hydrochloric acid and brine, dried over anhydrous sodium sulfate, and then concentrated under reduced pressure. The residue was purified by chromatography on a silica gel column, whereby the title compound was obtained as a colorless oil (11.21 g, yield: 78%).

### Example 1

### 1,4-Bis(5-phenyl-2-pyridyl)hexahydro-1,4-diazepine

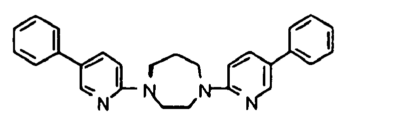

Under a nitrogen gas atmosphere, phenylboric acid (130.0 mg, 1.07 mmol), tetrakis(triphenylphosphine)palladium (56.0 mg, 0.050 mmol) and a 2.0 M aqueous solution of potassium carbonate (0.5 mL, 1.0 mmol) were added to a solution of the 1,4-bis(5-bromo-2-pyridyl)hexahydro-1,4-diazepine (200 mg, 0.585 mmol), which had been synthesized in Referential Example 1, in ethanol-toluene (2. 0 mL-2. 0 mL), and the resulting mixture was stirred at 80°C for 3 hours. After the reaction mixture was allowed cool down, the organic layer was collected and the water layer was extracted with chloroform. Those organic layers were combined, dried over anhydrous sodium sulfate, and then concentrated under reduced pressure. The residue was purified by chromatography on a silica gel column, and crude crystals were obtained. The crude crystals were recrystallized from chloroform-hexane, whereby the title compound was obtained as slightly yellow needles (melting point: 221.0-222.0°C) (61.0 mg, yield: 31%).
¹H-NMR (CDCl₃) δ: 2.17(tt, J=6.3, 6.3Hz, 2H), 3.65(dd, J=6.3, 6.3Hz, 4H), 3.96(s, 4H), 6.64(d, J=8.8Hz, 2H), 7.30(t, J=7.3Hz, 2H), 7.42(t, J=7.3Hz, 4H), 7.52(d, J=7.3Hz, 4H), 7.70(dd, J=2.4, 8.8Hz, 2H), 8.43(d, J=2.4Hz, 2H).

### Example 2

### 1,4-Bis[5-(2-methoxyphenyl)-2-pyridyl]hexahydro-1,4-diazepine

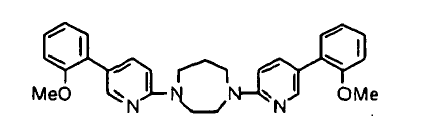

Following the procedures of Example 1, crude crystals were obtained from 1,4-bis(5-bromo-2-pyridyl)hexahydro-1,4-diazepine (206.0 mg, 0.500 mmol) synthesized in Referential Example 1 and 2-methoxyphenylboric acid (182.0 mg, 1.20 mmol). The crude crystals were recrystallized from methylene chloride-hexane, whereby the title compound was obtained as slightly yellow needles (melting point: 161.0-162.0°C) (215.0 mg, yield: 92%).
¹H-NMR (CDCl₃) δ: 2.16(tt, J=6.0, 6.0Hz, 2H), 3.64(dd, J=6.0, 6.0Hz, 4H), 3.83(s, 6H), 3.95(s, 4H), 6.60(d, J=8.7Hz,2 H), 6.65-7.06(m, 4H), 7.28-7.33(m, 4H), 7.69(dd, J=2.4, 8.7Hz, 2H), 8.36(d, J=2.4Hz, 2H).

### Example 3

### 1,4-Bis[5-(3-methoxyphenyl)-2-pyridyl]hexahydro-1,4-diazepine

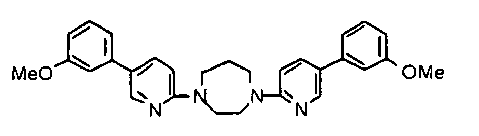

Following the procedures of Example 1, crude crystals were obtained from 1,4-bis(5-bromo-2-pyridyl)hexahydro-1,4-diazepine (206.0 mg, 0.500 mmol) synthesized in Referential Example 1 and 3-methoxyphenylboric acid (182.0 mg, 1.20 mmol). The crude crystals were recrystallized from methylene chloride-hexane, whereby the title compound was obtained as slightly yellow scales (melting point: 146.0-149.0°C) (121.0 mg, yield: 52%).
¹H-NMR (CDCl₃) δ: 2.16(tt, J=6.0, 6.0Hz, 2H), 3.63(dd, J=6.0, 6.0Hz, 4H), 3.85(s, 6H), 3.95(s, 4H), 6.62(d, J=8.7Hz, 2H), 6.84(ddd, J=1.9, 2.4, 8.0Hz, 2H), 7.05(dd, J=1.9, 2.4Hz, 2H), 7.15(ddd, J=2.4, 2.4, 7.8Hz, 2H), 7.33(dd, J=7.8, 8.0Hz, 2H), 7.68(dd, J=2.4, 8.7Hz, 2H), 8. 42 (d, J=2.4Hz, 2H).

### Example 4

### 1,4-Bis[5-(4-methoxyphenyl)-2-pyridyl]hexahydro-1,4-diazepine

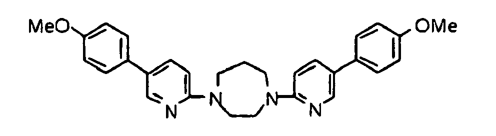

Following the procedures of Example 1, crude crystals were obtained from 1,4-bis(5-bromo-2-pyridyl)hexahydro-1,4-diazepine (206.0 mg, 0.500 mmol) synthesized in Referential Example 1 and 4-methoxyphenylboric acid (182.0 mg, 1.20 mmol). The crude crystals were recrystallized from chloroform-hexane, whereby the title compound was obtained as pale yellow needles (melting point: 259.0-256.0°C)(98.0 mg, yield: 42%).
¹H-NMR (CDCl₃) δ: 2.16(tt, J=6.0, 6.0Hz, 2H), 3.62(dd, J=6.0, 6.0Hz, 4H), 3.84(s, 6H), 3.94(s, 4H), 6.61(d, J=8.7Hz, 2H), 6.96(d, J=8.7Hz, 4H), 7.43(d, J=8.7Hz, 4H), 7.64(dd, J=2.4, 8.7Hz, 2H), 8.37(d, J=2.4Hz, 2H).

### Example 5

### 1,4-Bis[5-(4-methylthiophenyl)-2-pyridyl]hexahydro-1,4-diazepine

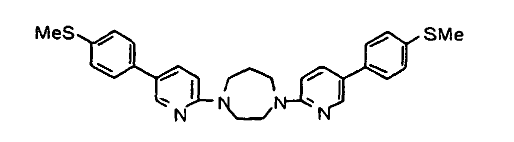

Following the procedures of Example 1, crude crystals were obtained from the 1,4-bis(5-bromo-2-pyridyl)hexahydro-1,4-diazepine (206.0 mg, 0.500 mmol) synthesized in Referential Example 1 and 4-methylthiophenylboric acid (202.0 mg, 1.20 mmol). The crude crystals were recrystallized from chloroform-hexane, whereby the title compound was obtained as pale yellow needles (melting point: 252.0-255.0°C)(109.0 mg, yield: 43%).
¹H-NMR (CDCl₃) δ: 2.16(tt, J=6.0, 6.0Hz, 2H), 2.51(s, 6H), 3.63(dd, J=6.0, 6.0Hz, 4H), 3.94(s, 4H), 6.61(d, J=8.7Hz, 2H), 7.31(d, J=8.2Hz, 4H), 7.43(d, J=8.2Hz, 4H), 7.66(dd, J=2.4, 8.7Hz, 2H), 8.40 (d, J=2.4Hz, 2H).

### Example 6

### 1,4-Bis[5-(4-methylphenyl)-2-pyridyl]hexahydro-1,4-diazepine

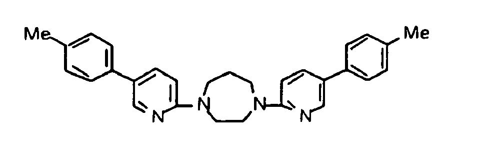

Following the procedures of Example 1, crude crystals were obtained from the 1,4-bis(5-bromo-2-pyridyl)hexahydro-1,4-diazepine (206.0 mg, 0.500 mmol) synthesized in Referential Example 1 and 4-methylphenylboric acid (163.0 mg, 1.20 mmol). The crude crystals were recrystallized from chloroform-hexane, whereby the title compound was obtained as pale yellow needles (melting point: 245.0-247.0°C) (93.0 mg, yield: 42%).
¹H-NMR (CDCl₃) δ: 2.16(tt, J=6.0, 6.0Hz, 2H), 2.38 (s, 6H), 3.63 (dd, J=6.0, 6.0Hz, 4H), 3.94 (s, 4H), 6.62(d, J=8.7Hz, 2H), 7.22 (d, J=8.0Hz, 4H), 7.41(d, J=8.0Hz, 4H), 7.67(dd, J=2.4, 8.7Hz, 2H), 8.40 (d, J=2.4Hz, 2H).

### Example 7

### 1,4-Bis[5-(4-t-butylphenyl)-2-pyridyl]hexahydro-1,4-diazepine

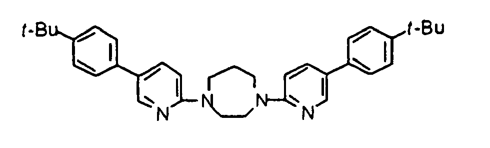

Following the procedures of Example 1, crude crystals were obtained from the 1,4-bis(5-bromo-2-pyridyl)hexahydro-1,4-diazepine (206.0 mg, 0.500 mmol) synthesized in Referential Example 1 and 4-t-butylphenylboric acid (226.0 mg, 1.20 mmol). The crude crystals were recrystallized from chloroform-hexane, whereby the title compound was obtained as colorless needles (melting point: 268.0-270.0°C) (117.0 mg, yield: 42%).
¹H-NMR (CDCl₃) δ: 1.35(s, 18H), 2.17(tt, J=6.0, 6.0Hz, 2H), 3.64(dd, J=6.0, 6.0Hz, 4H), 3.96(s, 4H), 6.63(d, J=8.7Hz, 2H), 7.45(s, 8H), 7.67(dd, J=2.4, 8.7Hz, 2H), 8.42(d, J=2.4Hz, 2H).

### Example 8

### 1,9-Bis[5-(9-trifluoromethylphenyl)-2-pyridyl)-hexahydro-1,4-diazepine

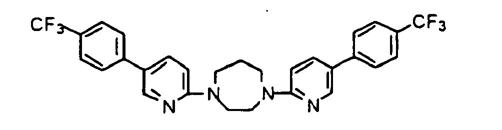

Following the procedures of Example 1, crude crystals were obtained from the 1,4-bis(5-bromo-2-pyridyl)hexahydro-1,4-diazepine (206.0 mg, 0.500 mmol) synthesized in Referential Example 1 and 4-trifluoromethylphenylboric acid (227.0 mg, 1.20 mmol). The crude crystals were recrystallized from chloroform-hexane, whereby the title compound was obtained as colorless needles (melting point: 236.0-238.0°C) (110.0 mg, yield: 40%).
¹H-NMR (CDCl₃) δ: 2.16(tt, J=6.0, 6.0Hz, 2H), 3.66(dd, J=6.0, 6.0Hz, 4H), 3.97(s, 4H), 6.65(d, J=8.7Hz, 2H), 7.60(d, J=8.5Hz, 4H), 7.66(d, J=8.5Hz, 4H), 7.70(dd, J=2.4, 8.7Hz, 2H), 8.43(d, J=2.4Hz, 2H).

### Example 9

### 1,4-Bis[5-(4-fluorophenyl)-2-pyridyl]hexahydro-1,4-diazepine

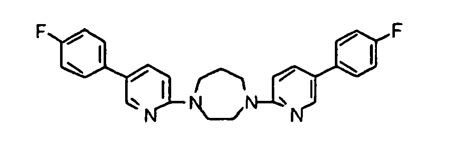

Following the procedures of Example 1, crude crystals were obtained from 1,4-bis(5-bromo-2-pyridyl)hexahydro-1,4-diazepine (206.0 mg, 0.500 mmol) synthesized in Referential Example 1 and 4-fluorophenylboric acid (168.0 mg, 1.20 mmol). The crude crystals were recrystallized from chloroform-hexane, whereby the title compound was obtained as colorless needles (melting point: 268.0-270.0°C) (115.0 mg, yield: 52%).
¹H-NMR (CDCl₃) δ: 2.16 (tt, J=6.0, 6.0Hz, 2H), 3.64(dd, J=6.0, 6.0Hz, 4H), 3.95(s, 4H), 6. 62 (d, J=8.7Hz, 2H), 7.10(dd, J=8.7Hz, ³J_{HF}=8.7Hz, 4H), 7.45(dd, J=8.7Hz, ⁴J_{HF}=5.3Hz, 4H), 7.63(d, J=2.4, 8.7Hz, 2H), 8.36(d, J=2.4Hz, 2H).

### Example 10

### 1,4-Bis[5-(4-chlorophenyl)-2-pyridyl]hexahydro-1,4-diazepine

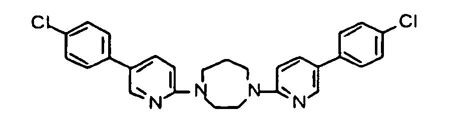

Following the procedures of Example 1, crude crystals were obtained from 1,4-bis(5-bromo-2-pyridyl)hexahydro-1,4-diazepine (206.0 mg, 0.500 mmol) and 4-chlorophenylboric acid (188.0 mg, 1.20 mmol). The crude crystals were recrystallized from chloroform-hexane, whereby the title compound was obtained as colorless needles (melting point: 245.0-247.0°C) (105.0 mg, yield: 44%).
¹H-NMR (CDCl₃) δ: 2.15(tt, J=6.0, 6.0Hz, 2H), 3.63(dd, J=6.0, 6.0Hz, 4H), 3.95(s, 4H), 6.62(d, J=8.7Hz, 2H), 7.37(d, J=8.2Hz, 4H), 7.43(d, J=8.2Hz, 4H), 7.64(dd, J=2.4, 8.7Hz, 2H), 8.38 (d, J=2.4Hz, 2H).

### Example 11

### 1,4-Bis[5-(2,3-dimethoxyphenyl)-2-pyridyl]hexahydro-1,4-diazepine dimethanesulfonate

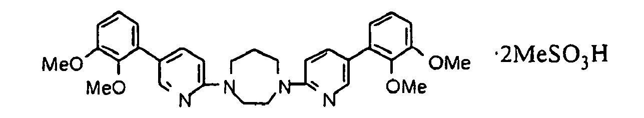

Following the procedures of Example 1, 1,4-bis[5-(2,3-dimethoxyphenyl)-2-pyridyl]hexahydro-1,4-diazepine was obtained as a colorless oil (28.0 mg, yield: 26%) from 1,4-bis(5-bromo-2-pyridyl)hexahydro-1,4-diazepine (82.0 mg,0.200 mmol) synthesized in Referential Example 1 and 2,3-dimethoxyphenylboric acid [Bull. Soc. Chim. Fr., 767-769 (1973)] (146.0 mg, 0.800 mmol) synthesized in a similar manner as in Referential Example 2.

To a solution of 1,4-bis[5-(2,3-dimethoxyphenyl)-2-pyridyl]hexahydro-1,4-diazepine (28.0 mg)(0.053 mmol) in chloroform (3.0 mL), a 1.0 M methanol solution of methane-sulfonic acid (0.11 mL, 0.11 mmol) was added, and the reaction mixture was concentrated under reduced pressure. The residue was recrystallized from methylene chloride-methanol-diethyl ether, whereby the title compound was obtained as pale brown crystalline powder (melting point: 199.5-202.0°C) (32.3 mg, yield: 86%).
¹H-NMR (CDCl₃) (ammonium salt NH⁺ protons were not observed) δ: 1.85-2.43(m, 2H), 2.90(s, 6H), 3.69(s, 6H), 3.90(s, 6H), 3.95-4.10(m, 4H), 4.31(s, 4H), 6.88(br.d, J=6.0Hz, 2H), 6.97(br.d, J=6.0Hz, 2H), 7.11 (br.dd, J=6.0Hz, 6.0Hz, 2H), 7.26(br.d, J=7.6Hz, 2H), 8.20(br.d, J=7.6Hz, 2H), 8.35(br.s, 2H).

### Example 12

### 1,4-Bis[5-(2,4-dimethoxyphenyl)-2-pyridyl]hexahydro-1,4-diazepine

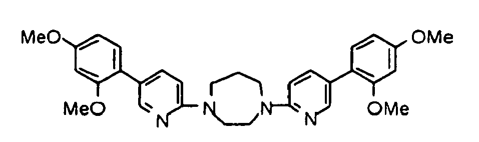

Following the procedures of Example 1, crude crystals were obtained from 1,4-bis(5-bromo-2-pyridyl)hexahydro-1,4-diazepine (206.0 mg,0.500 mmol) synthesized in Referential Example 1 and 2,4-dimethoxyphenylboric acid (218.0 mg, 1.20 mmol). The crude crystals were recrystallized from chloroform-hexane, whereby the title compound was obtained as colorless needles (melting point: 161.0-163.09C) (206.0 mg, yield: 78%).
¹H-NMR (CDCl₃) δ: 2.15 (tt, J=6.0, 6.0Hz, 2H), 3.63(dd, J=6.0, 6.0Hz, 4H), 3.80(s, 6H), 3.84(s, 6H), 3.93(s, 4H), 6.54-6.57(m, 4H), 6.58(d, J=8.7Hz, 2H), 7.21(d, J=9.0Hz, 2H), 7.64(dd, J=2.4, 8.7Hz, 2H), 8.30(d, J=2.4Hz, 2H).

### Example 13

### 1,4-Bis[5-(2,6-dimethoxyphenyl)-2-pyridyl]hexahydro-1,4-diazepine

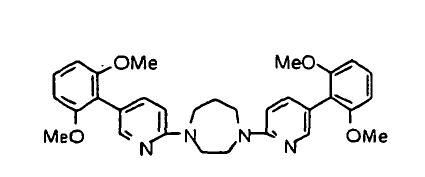

Following the procedures of Example 1, an oil was obtained from 1,4-bis(5-bromo-2-pyridyl)hexahydro-1,4-diazepine (100.0 mg, 0.240 mmol) synthesized in Referential Example 1 and 2,4-dimethoxyphenylboric acid (Chem. Abs. 123, pr256679r.) (150.0 mg, 1.00 mmol). Methanol-chloroform-hexane was added to the oil, and the resulting precipitate was collected by filtration, whereby the title compound was obtained as pale yellow crystalline powder (melting point: ≥300.0°C) (24.0 mg, yield: 19%).
¹H-NMR [CD₃-CDCl₃(1:19)] δ: 2.16(tt, J=6.1, 6.1Hz, 2H), 3.67(dd, J=6.1, 6.1Hz, 4H), 3.77(s, 12H), 3.92(s, 4H), 6.64(d, J=8.7Hz, 2H), 6.66(d, J=8.5Hz, 4H), 7.26(t, J=8.5Hz, 2H), 7.54 (dd, J=2.2, 8.7Hz, 2H), 8.15(d, J=2.2Hz, 2H).

### Example 14

### 1,4-Bis[5-(3,4-dimethoxyphenyl)-2-pyridyl]hexahydro-1,4-diazepine

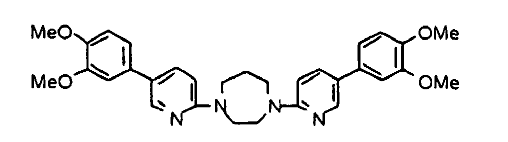

Following the procedures of Example 1, crude crystals were obtained from 1,4-bis(5-bromo-2-pyridyl)hexahydro-1,4-diazepine (206.0 mg, 0.500 mmol) synthesized in Referential Example 1 and 3,4-dimethoxyphenylboric acid (218.0 mg, 1.20 mmol). The crude crystals were recrystallized from chloroform-hexane, whereby the title compound was obtained as pale yellow needles (melting point: 190.0-192.0°C) (129.0 mg, yield: 48%).
¹H-NMR (CDCl₃) δ: 2.16 (tt, J=6.0, 6.0Hz, 2H), 3.63(dd, J=6.0, 6.0Hz, 4H), 3.91(s, 6H), 3.93(s, 6H), 3.95(s, 4H), 6.62(d, J=8.7Hz, 2H), 6.93(d, J=8.2Hz, 2H), 7.02(d, J=1.9Hz, 2H), 7.05(dd, J=1.9, 8.2Hz, 2H), 7.65(dd, J=2.4, 8.7Hz, 2H), 8.39(d, J=2.4Hz, 2H).

### Example 15

### 1,4-Bis[5-(3,9-methylenedioxyphenyl)-2-pyridyl]-hexahydro-1,4-diazepine

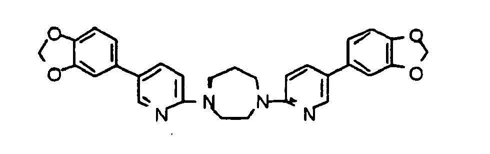

Following the procedures of Example 1, crude crystals were obtained from 1,4-bis(5-bromo-2-pyridyl)hexahydro-1,4-diazepine (206.0 mg,0.500 mmol) synthesized in Referential Example 1 and 3,4-methylenedioxyphenylboric acid (199.0 mg, 1.20 mmol). The crude crystals were recrystallized from chloroform-hexane, whereby the title compound was obtained as pale yellow needles (melting point: 235.0-237.0°C) (105.0 mg, yield: 42%).
¹H-NMR (CDCl₃) δ: 2.15(tt, J=6.0, 6.0Hz, 2H), 3.62(dd, J=6.0, 6.0Hz, 4H), 3.93(s, 4H), 5.98(s, 4H), 6.59(d, J=8.7Hz, 2H), 6.86(d, J=8.0Hz, 2H), 6.95(dd, J=1.7, 8.0Hz, 2H), 6.98(d, J=1.7Hz, 2H), 7.60(dd, J=2.4, 8.7Hz, 2H), 8.32(d, J=2.4Hz, 2H).

### Example 16

### 1,4-Bis[5-(3,5-dimethoxyphenyl)-2-pyridyl]hexahydro-1,4-diazepine dimethanesulfonate

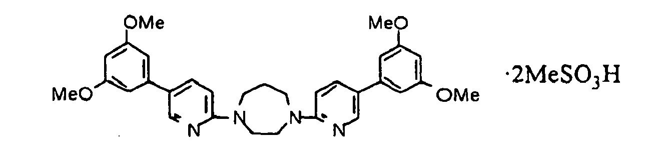

Following the procedures of Example 1, 1,4-bis[5-(3,5-dimethoxyphenyl)-2-pyridyl]hexahydro-1,4-diazepine was obtained as a colorless oil (89.0 mg, yield: 71%) from 1,4-bis(5-bromo-2-pyridyl)hexahydro-1,4-diazepine (100.0 mg, 0.240 mmol) synthesized in Referential Example 1 and 3, 5-dimethoxyphenylboric acid (79. 0 mg, 0.530 mmol) synthesized in a similar manner as in Referential Example 2.

To a solution of 1,4-bis[5-(3,5-dimethoxyphenyl)-2-pyridyl]hexahydro-1,4-diazepine (89.0mg) (0.170 mmol) inmethanol (5.0mL), a 1.0M aqueous solution of methanesulfonic acid (0. 34 mL, 0.34 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as pale yellow crystalline powder (melting point: 181.0-183.0°C) (81.0mg, yield: 66%).
¹H-NMR (CDCl₃) (ammonium salt NH⁺ protons were not observed) δ: 2.35-2.45(m, 2H), 2.91(s, 6H), 3.81(s, 12H), 3.95-4.10(m, 4H), 4.31(s, 4H), 6.47(t, J=2.2Hz, 2H), 6.55(d, J=2.2Hz, 4H), 7.34(d, J=9.6Hz, 2H), 8.12(dd, J=2.2, 9.6Hz, 2H), 8.36(d, J=2.2Hz, 2H).

### Example 17

### 1,4-Bis[5-(3,5-diethoxyphenyl)-2-pyridyl]hexahydro-1,4-diazepine

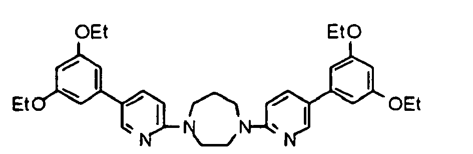

Under a nitrogen gas atmosphere, bis(picolato)diboron (223.0 mg, 0.880 mmol), dichloro[1,1'-bis(diphenyl-phosphino)ferrocene]palladium(II) dichloromethane complex (1:1) (19.6 mg, 0.024 mmol), 1,1'-bis(diphenylphosphino)-ferrocene (13.3 mg, 0.024 mmol) and potassium acetate (236.0 mg, 2.40 mmol) were added to a solution of 3,5-diethoxyphenyl trifluoromethanesulfonate (WO99/41224) (251.0 mg, 0.800 mmol) in dimethylformamide (6.5 mL), and the resulting mixture was stirred at 80°C for 12 hours. To the reaction mixture, 1,4-bis(5-bromo-2-pyridyl)hexahydro-1,4-diazepine (110.0 mg, 0.270 mmol) synthesized by the process described in Referential Example 1, dichloro[1,1'-bis(diphenylphosphino)ferrocene]-palladium(II) dichloro-methane complex (1:1) (19.6 mg, 0.024 mmol) and a 2.0 M aqueous solution of sodium carbonate (2.0 mL, 4.0 mmol) were added. The resulting mixture was stirred at 80°C for 1 hour. Water was added to the reaction mixture, followed by extraction with diethyl ether. The organic layer was dried over anhydrous sodium sulfate and then concentrated under reduced pressure. The residue was purifiedby chromatography on a silica gel column, whereby the title compound was obtained as colorless amorphous powder (70.0 mg, yield: 45%).
¹H-NMR (CDCl₃) δ: 1.43(t, J=7.0Hz, 12H), 2.16(tt, J=6.2, 6.2Hz, 2H), 3.63(dd, J=6.2, 6.2Hz, 4H), 3.94(s, 4H), 4.06(q, J=7.0Hz, 8H), 6.40(t, J=2.2Hz, 2H), 6.60(d, J=8.9Hz, 2H), 6.64(d, J=2. 2Hz, 4H), 7.66(dd, J=2.6, 8.9Hz, 2H), 8.41(d, J=2.6Hz, 2H).

### Example 18

### 1,4-Bis[5-(3,5-diisopropoxyphenyl)-2-pyridyl]hexahydro-1,4-diazepine

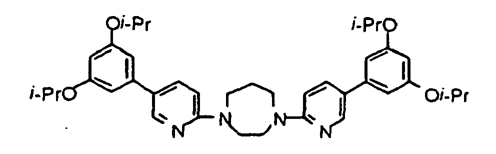

Following the procedures of Example 17, an oil was obtained from 1,4-bis(5-bromo-2-pyridyl)hexahydro-1,4-diazepine (103.0 mg, 0.250 mmol) synthesized in Referential Example 1 and 3,5-diisopropoxyphenyl trifluoromethane-sulfonate (261.0 mg, 0.760 mmol) synthesized in Referential Example 3. Hexane was added to the oil, and the resulting precipitate was collected by filtration, whereby the title compound was obtained as colorless crystalline powder (melting point: 131.0-132.01C) (78.0 mg, yield: 49%).
¹H-NMR (CDCl₃) δ: 1.36(d, J=6.0Hz, 24H), 2.16(tt, J=6.2, 6.2Hz, 2H), 3.63(dd, J=6.2, 6.2Hz, 4H), 3.94(s, 4H), 4.57(qq, J=6.0, 6.0Hz, 4H), 6.39(t, J=2.3Hz, 2H), 6.60(d, J=8.9Hz, 2H), 6.62(d, J=2.3Hz, 4H), 7.66(dd, J=2.6, 8.9Hz, 2H), 8.40(d, J=2.6Hz, 2H).

### Example 19

### 1,4-Bis[5-(3,5-dimethylphenyl)-2-pyridyl]hexahydro-1,4-diazepine dimethanesulfonate

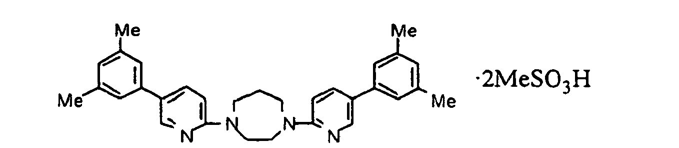

Following the procedures of Example 1, 1,4-bis[5-(3,5-dimethylphenyl)-2-pyridyl]hexahydro-1,4-diazepine was obtained as yellow crystalline powder (83.0 mg, yield: 89%) from 1,4-bis(5-bromo-2-pyridyl)hexahydro-1,4-diazepine (82.0 mg, 0.200 mmol) synthesized in Referential Example 1 and 3,5-dimethylphenylboric acid (90.0 mg, 0.600 mmol).

To a solution of 1,4-bis[5-(3,5-dimethylphenyl)-2-pyridyl]hexahydro-1,4-diazepine (83.0 mg) (0.170 mmol) in methanol (5.0 mL), a 1.0 M aqueous solution of methanesulfonic acid (0.37 mL, 0.37 mmol) was added, and the resulting mixture was concentrated under reduced pressure. Ethanol (5.0 mL) was added to the residue, and the resulting mixture was concentrated under reduced pressure. The residue was recrystallized from ethanol-diethyl ether-hexane, whereby the title compound was obtained as colorless needles (melting point: 188.0°C (decomposed)(101.0 mg, yield: 86%).
¹H-NMR (DMSO-d₆, 120°C) (ammonium salt NH⁺ protons were not observed) δ: 2.02(tt, J=5.8, 5.8Hz, 2H), 2.28(s, 12H), 2.46(s, 6H), 3.78(dd, J=5.8, 5.8Hz, 4H), 4.01(s, 4H), 6.94(br.s, 2H), 6.99(d, J=9.2Hz, 2H), 7.09(br.s, 4H), 7.88(dd, J=2.4, 9.2Hz, 2H), 8.19(d, J=2.4Hz, 2H).

### Example 20

### 1,4-Bis[5-[3,5-bis(methoxycarbonyl)phenyl]-2-pyridyl]-hexahydro-1,4-diazepine

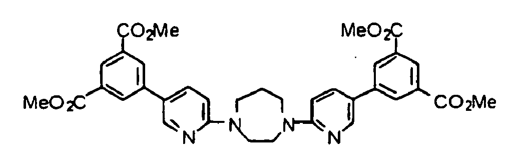

Following the procedures of Example 17, an oil was obtained from 1,4-bis(5-bromo-2-pyridyl)hexahydro-1,4-diazepine (207. 8 mg, 0.504 mmol) synthesized in Referential Example 1 and 3,5-bis(methoxycarbonyl)phenyl trifluoro-methanesulfonate [J. Org. Chem., 65, 5360-5370 (2000)] (474.4 mg, 1.39 mmol). Diethyl ether was added to the oil, and the resulting precipitate was collected by filtration, whereby the title compound was obtained as colorless crystalline powder (melting point: 216.0-217.5°C)(92.8 mg, yield: 10%).
¹H-NMR (CDCl₃) δ: 2.17(tt, J=6.1, 6.1Hz, 2H), 3.66(dd, J=6.1, 6.1Hz, 4H), 3.97(s, 12H), 3.98(s, 4H), 6.66(d, J=8.9Hz, 2H), 7.76(dd, J=2.5, 8.9Hz, 2H), 8.38(d, J=1.1Hz, 4H), 8.49(d, J=2.5Hz, 2H), 8.58 (t, J=1.1Hz, 2H).

### Example 21

### 1,4-Bis[5-[3,5-bis(hydroxymethyl)phenyl]-2-pyridyl]-hexahydro-1,4-diazepine

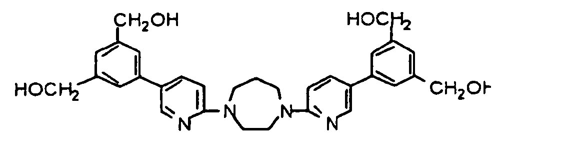

Under a nitrogen gas atmosphere, a solution of 1,4-bis[5-3[3,5-bis(methoxycarbonyl)phenyl]-2-pyridyl]-hexahydro-1,4-diazepine (17.0 mg, 0.0266 mmol), which had been synthesized in Example 20, in anhydrous tetrahydrofuran (1.0 mL) was cooled to -20°C. To the resulting solution, a 1.0 M toluene solution of diisobutylaluminum hydride (0.40 mL, 0.40 mmol) was added, and the resulting mixture was stirred for 1 hour. Methanol (0.2 mL, 4.9 mmol) was added to the mixture. A bath was removed, followed by the addition of 0.50M hydrochloric acid (1.0 mL, 0.50 mmol). The thus-obtained mixture was stirred at room temperature for 1 hour, to which a 1.0 M aqueous solution of sodium hydroxide (1.0 mL, 1.0 mmol) and brine were added. The resulting mixture was extracted with chloroform, and the organic layer was dried over anhydrous sodium sulfate and then concentrated under reduced pressure. The residue was purified by preparative thin-layer chromatography on silica gel, whereby the title compound was obtained as colorless amorphous powder (7.4 mg, yield: 53%).
¹H-NMR (CD₃OD) δ: 2.31(tt, J=5.9, 5.9Hz, 2H), 3.86(dd, J=5.9, 5.9Hz, 4H), 4.14(s, 4H), 4.83(s, 8H), 6.98(d, J=8.9Hz, 2H), 7.46(br.s, 2H), 7.60(br.s, 4H), 7.97(dd, J=2.3, 8.9Hz, 2H), 8.51(d, J=2.3Hz, 2H).

### Example 22

### 1,4-Bis[5-[3,5-bis(methoxymethyl)phenyl]-2-pyridyl]-hexahydro-1,4-diazepine dihydrochloride

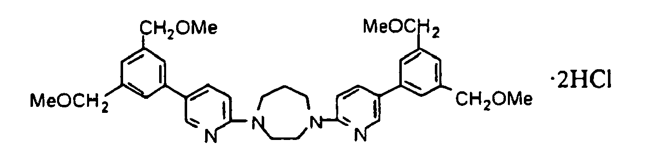

Following the procedures of Example 17, 1,4-bis[5-[3,5-bis(methoxymethyl)-phenyl]-2-pyridyl]hexahydro-1,4-diazepine was obtained as a colorless oil (51.9 mg, yield: 33%) from 1,4-bis(5-bromo-2-pyridyl)hexahydro-1,4-diazepine (99.5 mg, 0.242 mmol) synthesized in Referential Example 1 and 3,5-bis(methoxymethyl)phenyl trifluoro-methanesulfonate (171.0 mg, 0.545 mmol) synthesized in Referential Example 4.

To a solution of 1,4-bis[5-[3,5-bis(methoxymethyl)-phenyl]-2-pyridyl] hexahydro-1,4-diazepine (51.9 mg) (0.0892 mmol) in ethanol (5.0 mL), 1.0 M hydrochloric acid (0.21 mL, 0.21 mmol) was added, and the reaction mixture was concentrated under reduced pressure. Ethanol (5.0 mL) was added to the residue and the resulting mixture was concentrated under reduced pressure, whereby the title compound was obtained as colorless amorphous powder(47.8 mg, yield: 92%).
¹H-NMR (CDCl₃) (ammonium salt NH⁺ protons were not observed) δ: 2.36-2.50(m, 2H), 3.42(br.s, 12H), 4.02-4.22(m, 4H), 3.96-4.52(m, 4H), 4.48(br.s, 8H), 7.14-7.32(m, 2H), 7.33(br.s, 2H), 7.39(br.s, 4H), 8.09-8.12(m, 2H), 8.38-8.44(m, 2H).

### Example 23

### 1,4-Bis[5-(2,4,6-trimethoxyphenyl)-2-pyridyl]-hexahydro-1,4-diazepine

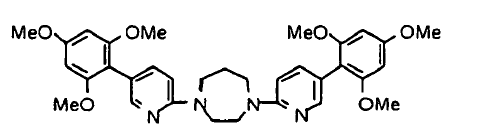

Following the procedures of Example 1, an oil was obtained from 1,4-bis(5-bromo-2-pyridyl)hexahydro-1,4-diazepine (120.0 mg, 0.290 mmol) synthesized in Referential Example 1 and 2,4,6-trimethoxyphenylboric acid [Tetrahedron Lett., 32,2229-2232 (1991)] (304.0 mg, 1.45 mmol). Chloroform-hexane was added to the oil, and the resulting precipitate was collected by filtration, whereby the title compound was obtained as yellow crystalline powder (melting point: 243.0-247.0°C) (29.0 mg, yield: 17%).
¹H-NMR (CDCl₃) δ: 2.16(tt, J=6.1, 6.1Hz, 2H), 3.65(dd, J=6.1, 6.1Hz, 4H), 3.75(s, 12H), 3.86(s, 6H), 3.93(s, 4H), 6.23(s, 4H), 6.59(d, J=8.9Hz, 2H), 7.48(dd, J=2.2, 8.8Hz, 2H), 8.17(d, J=2.2Hz, 2H).

### Example 24

### 1,4-Bis[5-(2,3,4-trimethoxyphenyl)-2-pyridyl]-hexahydro-1,4-diazepine

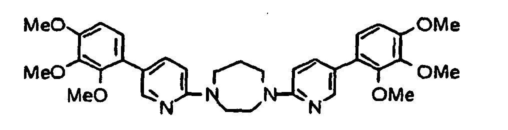

Following the procedures of Example 1, an oil was obtained from 1,4-bis(5-bromo-2-pyridyl)hexahydro-1,4-diazepine (115.0 mg, 0.280 mmol) synthesized in Referential Example 1 and 2,3,4-trimethoxyphenylboric acid (130.0 mg, 0.620 mmol) synthesized from 2,3,4-trimethoxybromobenzne [J. Org. Chem., 23, 16-17 (1958)] in a similar manner as in Referential Example 2. Chloroform-hexane was added to the oil, and the resulting precipitate was collected by filtration, whereby the title compound was obtained as yellow crystalline powder (melting point: 192.0-194.0°C) (57.0 mg, yield: 35%).
¹H-NMR (CDCl₃) δ: 2.17(tt, J=6.1, 6.1Hz, 2H), 3.65(dd, J=6.1, 6.1Hz, 4H), 3.72(s, 6H), 3.89(s, 6H), 3.92(s, 6H), 3.96(s, 4H), 6.60(d, J=8.8Hz, 2H), 6.73(d, J=8.5Hz, 2H), 7.00(d, J=8.5Hz, 2H), 7.67(dd, J=2.4, 8.8Hz, 2H), 8.28(d, J=2.4Hz, 2H).

### Example 25

### 1,4-Bis[5-(2,3,5-trimethoxyphenyl)-2-pyridyl]-hexahydro-1,4-diazepine

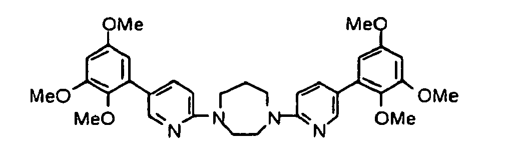

Following the procedures of Example 1, an oil was obtained from 1,4-bis(5-bromo-2-pyridyl)hexahydro-1,4-diazepine (109.0 mg, 0.264 mmol) synthesized in Referential Example 1 and 2,3,5-trimethoxyphenylboric acid (140.0 mg, 0.660 mmol) synthesized from 1-bromo-2,3,5-trimethoxybenzene [J. Am. Chem., 61, 144-147 (1939)] in a similar manner as in Referential Example 2. Methanol-diethyl ether was added to the oil, and the resulting precipitate was collected by filtration, whereby the title compound was obtained as pale brown crystalline powder (melting point: 155.0-160.0°C) (15.0 mg, yield: 10%).
¹H-NMR (CDCl₃) δ: 2.13-2.23(m, 2H), 3.56(s, 6H), 3.64-3.70(m, 4H), 3.81(s, 6H), 3.88(s, 6H), 3.97(s, 4H), 6.43(d, J=2.7Hz, 2H), 6.48(d, J=2.7Hz, 2H), 6.62(d, J=9.0Hz, 2H), 7.76 (dd, J=2.2, 9.0Hz, 2H), 8.37(d, J=2.2Hz, 2H).

### Example 26

### 1,4-Bis[5-(3,4,5-trimethoxyphenyl)-2-pyridyl]hexahydro-1,4-diazepine dimethanesulfonate

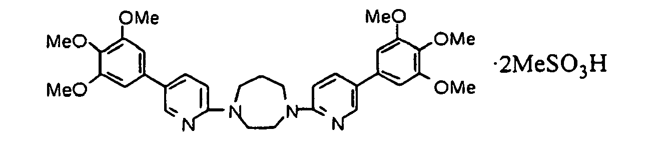

Following the procedures of Example 1, 1,4-bis[5-(3,4,5-trimethoxylphenyl)-2-pyridyl]-hexahydro-1,4-diazepine was obtained as a colorless oil (22.6 mg, yield: 53%) from 1,4-bis(5-bromo-2-pyridyl)hexahydro-1,4-diazepine (30.0 mg, 0.0730 mmol) synthesized in Referential Example 1 and 3,4,5-trimethoxylphenylboric acid (45.4 mg, 0.214 mmol).

To a solution of 1,4-bis[5-(3,4,5-trimethoxylphenyl)-2-pyridyl]hexahydro-1,4-diazepine (72.4 mg, 0.120 mmol) in ethanol-chloroform (1:3, 600 mL), methanesulfonic acid (24.9 g, 0.258 mmol) was added, and the reaction mixture was concentrated under reduced pressure. The concentration residue was recrystallized from methanol-diethyl ether, whereby the title compound was obtained as slightly yellow crystalline powder (melting point: 204.0-206.0°C (88.2 g, yield: 92%).
¹H-NMR (DMSO-d₆, 120°C) (ammonium salt NH⁺ protons were not observed) δ: 1.95-2.05(m, 2H), 2.42(s, 6H), 3.72(s, 6H), 3.74-3.77(m, 4H), 3.82(s, 12H), 3.98(s, 4H), 6.79(s, 4H), 6.92(d, J=9.0Hz, 2H), 7.88(dd, J=2.4, 9.0Hz, 2H), 8.26(d, J=2.4Hz, 2H).

### Example 27

### 1,4-Bis[5-(3,4,5-triethoxyphenyl)-2-pyridyl]hexahydro-1,4-diazepine dimethanesulfonate

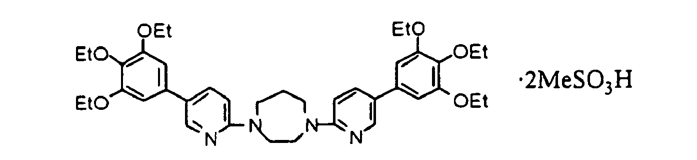

Following the procedures of Example 1, 1,4-bis[5-(3,4,5-triethoxylphenyl)-2-pyridyl]hexahydro-1,4-diazepine was obtained as a colorless oil (77 mg, yield: 48%) from 1,4-bis(5-bromo-2-pyridyl)hexahydro-1,4-diazepine (100.0 mg, 0.240 mmol) synthesized in Referential Example 1 and 3,4,5-triethoxylphenylboric acid (134.0 mg, 0.530 mmol) synthesized in Referential Example 5.

To a solution of 1,4-bis[5-(3,4,5-triethoxylphenyl)-2-pyridyl]hexahydro-1,4-diazepine (77mg, 0.115mmol) inmethanol (5.0mL), a1.0Maqueous solution of methanesulfonic acid (0.23 mL, 0.23 mmol) was added, and the reaction mixture was concentrated under reduced pressure. Ethanol (5.0 mL) was added to the residue, and the resulting mixture was concentrated under reduced pressure. The residue was recrystallized from methanol-diethyl ether, whereby the title compound was obtained as pale yellow crystalline powder (melting point: 235.0-237.0°C (74.0 mg, yield: 94%).
H-NMR (DMSO-d₆,120°C) (ammonium salt NH⁺ protons were not observed) δ: 1.26(t, J=7.1Hz, 6H), 1.33(t, J=7.1Hz, 12H), 2.01(tt, J=5.9, 5.9Hz, 2H), 2.40(s, 6H), 3.74(dd, J=5.9, 5.9Hz, 4H), 3.96(s, 4H), 3.99(q, J=7.1Hz, 4H), 4.08 (q, J=7.1Hz, 8H), 6.75 (s, 4H), 6.88(d, J=9.5Hz, 2H), 7.83(dd, J=2.4, 9.5Hz, 2H), 8.23(d, J=2.4Hz, 2H).

### Example 28

### 1,4-Bis[5-(3,5-diisopropoxy-4-methoxyphenyl)-2-pyridyl]hexahydro-1,4-diazepine

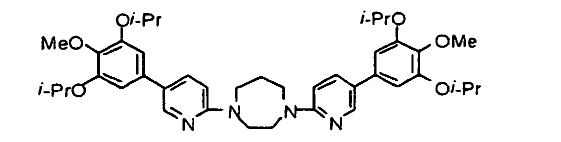

Following the procedures of Example 1, an oil was obtained from 1,4-bis(5-bromo-2-pyridyl)hexahydro-1,4-diazepine (100.0 mg, 0.240 mmol) synthesized in Referential Example 1 and 3,5-diisopropoxy-4-methoxyphenylboric acid (142.0 mg, 0.530 mmol) synthesized in Referential Example 6. The oil was recrystallized from chloroform-hexane, whereby the title compound was obtained as slightly brown needles (melting point: 164.0-165.0°C) (90.0 mg, yield: 53%).
¹H-NMR (CDCl₃) δ: 1.38(d, J=5.9Hz, 24H), 2.16(tt, J=5.9, 5.9Hz, 2H), 3.63(dd, J=5.9, 5.9Hz, 4H), 3.85(s, 6H), 3.95(s, 4H), 4.57(qq, J=5.9, 5.9Hz, 4H), 6.61(d, J=9.0Hz, 2H), 6.70(s, 4H), 7.63(dd, J=2.4, 9.0Hz, 2H), 8.37(d, J=2.4Hz, 2H).

### Example 29

### 1,4-Bis[5-(4-isopropoxy-3,5-dimethoxyphenyl)-2-pyridyl]hexahydro-1,4-diazepine dimethanesulfonate

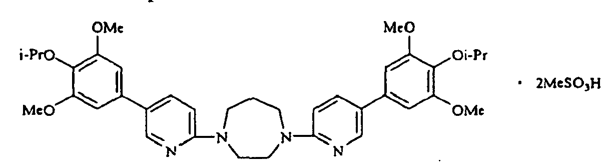

Following the procedures of Example 1, 1,4-bis[5-(4-isopropoxy-3.5-dimethoxyphenyl)-2-pyridyl]hexahydro-1-4-diazepine(107.0 mg, yield: 69%) was obtained as a sticky oil from 1,4-bis(5-bromo-2-pyridyl)hexahydro-1,4-diazepine (100.0 mg, 0.240 mmol) synthesized in Referential Example 1 and 4-isopropoxy-3,5-dimethoxyphenylboric acid (127.0 mg, 0.530 mmol) synthesized from methyl syringate in a similar manner as in Referential Example 6. A 1.0M aqueous solution of methanesulfonic acid ((0.35 mL, 0.35 mmol) was added to the oil (107.0 mg, 0.166 mmol) in methanol (5.0 mL), and the resulting mixture was concentrated under reduced pressure. A preciptate was collected by adding methanol-diethyl ether to the residue, whereby the title compound was obtained as brown crystalline powder (melting point: 192.0-196.0°C) (100.0 mg, yield: 72%).
¹H-NMR (DMSO-d₆, 120°C) (ammonium salt NH⁺ protons were not observed) δ: 1.21(d, J=6.1Hz, 12H), 2.02(tt, J=5.9, 5.9Hz, 2H), 2.41(s, 6H), 3.75(dd, J=5.9, 5.9Hz, 4H), 3.80(s, 12H), 3.97(s, 4H), 4.30(qq, J=6.1, 6.1Hz, 2H), 6.78(s, 4H), 6.90(d, J=9.3Hz, 2H), 7.87(dd, J=2.4, 9.3Hz, 2H), 8.27(d, J=2.4Hz, 2H).

### Example 30

### 1,4-Bis[5-(3-methoxy-4,5-methylenedioxyphenyl)-2-pyridyl]hexahydro-1,4-diazepine dimethanesulfonate

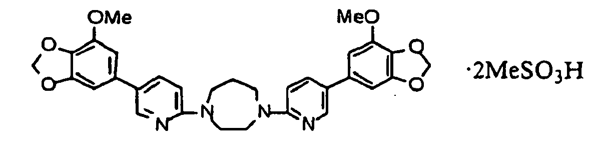

Following the procedures of Example 1, 1,4-bis[5-(3-methoxy-4,5-methylenedioxyphenyl)-2-pyridyl]-hexahydro-1,4-diazepine was obtained as a colorless oil (92.0 mg, yield: 71%)from 1,4-bis(5-bromo-2-pyridyl)hexahydro-1,4-diazepine (100.0 mg, 0.240 mmol) synthesized in Referential Example 1 and 3-methoxy-4,5-methylenedioxyphenylboric acid (103.0 mg, 0.530 mmol) synthesized in Referential Example 7.

To a solution of 1,4-bis[5-(3-methoxy-4,5-methylene-dioxyphenyl)-2-pyridyl]hexahydro-1,4-diazepine (92.0 mg, 0.170 mmol) in methanol (5.0 mL), a 1.0 M aqueous solution of methanesulfonic acid (0.33 mL, 0. 33 mmol) was added, and the reaction mixture was concentrated under reduced pressure. Ethanol (5.0mL) was added to the residue, and the resulting mixture was concentrated under reduced pressure. Methanol-diethyl ether was added to the residue, and the resulting precipitate was collected, whereby the title compound was obtained as pale yellow crystalline powder (melting point: 212.0-215.0°C (87.0 mg, yield: 68%).
¹H-NMR (CDCl₃) (data of free base of the title compound) δ: 2.15(tt, J=6.1, 6.1Hz, 2H), 3.63(dd, J=6.1, 6.1Hz, 4H), 3.94(s, 4H), 3.95(s, 6H), 6.00(s, 4H), 6.60(d, J=8.8Hz, 2H), 6.65(s, 2H), 6.68(s, 2H), 7.60(dd, J=2.4, 8.8Hz, 2H), 8.34 (d, J=2.4Hz, 2H).

### Example 31

### 1,9-Bis[5-(9-hydroxy-3,5-dimethoxyphenyl)-2-pyridyl]hexahydro-1,4-diazepine

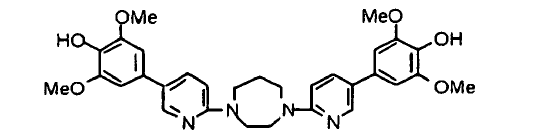

Following the procedures of Example 1, 1,4-bis[5-(4-benzyloxy-3,5-dimethoxyphenyl)-2-pyridyl]hexahydro-1,4-diazepine was obtained as a colorless oil (61.0 mg, yield: 63%) from 1,4-bis(5-bromo-2-pyridyl)hexahydro-1,4-diazepine (54.0 mg, 0.130 mmol) synthesized in Referential Example 1 and 4-benzyloxy-3,5-dimethoxyphenylboric acid (80.0 mg, 0.280 mmol) synthesized in Referential Example 8.

To a solution of 1,4-bis[5-(4-benzyloxy-3,5-dimethoxyphenyl)-2-pyridyl]hexahydro-1,4-diazepine (27.0 mg, 0.037 mmol) in methanol (1.0 mL), 10% palladium on charcoal (14.0 mg) and ammonium formate (14.0 mg, 0.220 mmol) were added, and the resulting mixture was stirred at 70°C for 2 hours. Insoluble matter was filtered off by using Celite, and the filtrate was concentrated under reduced pressure. A solution of the residue in chloroform was washed with water and brine, dried over anhydrous sodium sulfate, and then concentrated under reduced pressure. The residue was recrystallized frommethanol-chloroform-diethyl ether, whereby the title compound was obtained as pale brown needles (melting point: 200.0-202.0°C) (17.0 mg, yield: 85%).
¹H-NMR (CDCl₃) δ: 2.16(tt, J=6.3, 6.3Hz, 2H), 3.63(dd, J=6.3, 6.3Hz, 4H), 3.94(s, 12H), 3.95(s, 4H), 5.50(s, 2H), 6.61(d, J=8.8Hz, 2H), 6.71(s, 4H), 7.46(dd, J=2.2, 8.8Hz, 2H), 8.37(d, J=2.2Hz, 2H).

### Example 32

### 1,4-Bis[5-(3-hydroxy-4,5-dimethoxyphenyl)-2-pyridyl]hexahydro-1,4-diazepine dimethanesulfonate

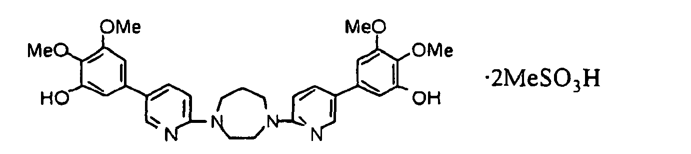

The procedures described in Example 31 were applied. From 1,4-bis(5-bromo-2-pyridyl)hexahydro-1,4-diazepine (115.0 mg, 0.280 mmol) synthesized in Referential Example 1 and 3-benzyloxy-4,5-dimethoxyphenylboric acid (170.0 mg, 0.590 mmol) synthesized frommethyl 3-hydroxy-4,5-dimethoxybenzoate [Indian J. Chem., 21B, 27-29, (1982)] by similar procedures as in Referential Example 8, 1,4-bis[5-(3-benzyloxy-4,5-dimethoxyphenyl)-2-pyridyl]hexahydro-1,4-diazepine was obtained as a colorless viscous oil (173.0 mg, yield: 84%). From 1,4-bis[5-(3-benzyloxy-4,5-dimethoxyphenyl)-2-pyridyl]hexahydro-1,4-diazepine (157.0 mg, 0.210 mmol), 1,9-bis[5-(3-hydroxy-4,5-dimethoxyphenyl)-2-pyridyl]hexahydro-1,4-diazepine was obtained as a colorless oil (97.0 mg, yield: 84%).

To a solution of 1,4-bis[5-(3-hydroxy-4,5-dimethoxy-phenyl)-2-pyridyl]hexahydro-1,4-diazepine (97.0 mg, 0.170 mmol) in methanol (5.0 mL), a 1.0 M aqueous solution of methanesulfonic acid (0.35 mL, 0.35 mmol) was added, and the reaction mixture was concentrated underreducedpressure. Ethanol (5.0mL) was added to the residue, and the resulting mixture was concentrated under reduced pressure. The residue was recrystallized from methanol, whereby the title compound was obtained as colorless crystalline powder (melting point: 300.0-301.0°C (84.0 mg, yield: 63%).
¹H-NMR (DMSO-d₆, 120°C) (neither ammonium salt NH⁺ protons nor phenol OH proton was observed) δ: 1.96-2.06(m, 2H), 2.41(s, 6H), 3.71-3.77(m, 4H), 3.73(s, 6H), 3.81(s, 6H), 3.97(s, 4H), 6.62(d, J=2.2Hz, 2H), 6.65(d, J=2.2Hz, 2H), 6.94(d, J=9.3Hz, 2H), 7.83(dd, J=2.4, 9.3Hz, 2H), 8.17(d, J=2.4Hz, 2H).

### Example 33

### 1,4-Bis[5-(3,5-dimethoxy-4-methylthiophenyl)-2-pyridyl]hexahydro-1,4-diazepine dimethanesulfonate

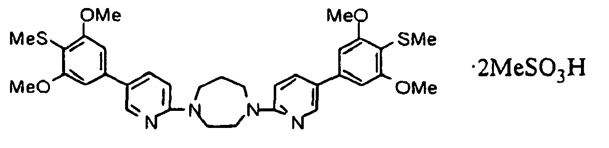

Following the procedures of Example 1, 1,4-bis[5-(3,5-dimethoxy-4-methylthiophenyl)-2-pyridyl]hexahydro-1,4-diazepine was obtained as a colorless oil (72.0 mg, yield: 61%) from 1,4-bis(5-bromo-2-pyridyl)hexahydro-1,4-diazepine (80.0 mg, 0.192 mmol) synthesized in Referential Example 1 and 3,5-dimethoxy-4-methylthiophenylboric acid (96.0 mg, 0.422 mmol) synthesized in Referential Example 9.

To a solution of 1,4-bis[5-(3,5-dimethoxy-4-methylthiophenyl)-2-pyridyl]hexahydro-1,4-diazepine (72.0 mg, 0.120 mmol) in methanol (5.0 mL), a 1.0 M aqueous solution of methanesulfonic acid (0.24 mL, 0.24 mmol) was added, and the reaction mixture was concentrated under reduced pressure. Ethanol (5.0 mL) was added to the residue, and the resulting mixture was concentrated under reduced pressure. Methanol-diethyl ether was added to the residue, and the resulting precipitate was collected, whereby the title compound was obtained as pale yellow crystalline powder (melting point: 212.0-217.0°C (58.0 mg, yield: 61%).
¹H-NMR (CDCl₃) (ammonium salt NH⁺ protons were not observed) δ: 2.37-2.40(m, 2H), 2.38(s, 6H), 2.91(s, 6H), 3.92(s, 12H), 4.00-4.05(m, 4H), 4.32(s, 4H), 6.61(s, 4H), 7.40(d, J=9.4Hz, 2H), 8.16(dd, J=2.2, 9.4Hz, 2H), 8.46 (d, J=2.2Hz, 2H).

### Example 34

### 1,9-Bis[5-(9-amino-3,5-dimethoxyphenyl)-2-pyridyl]hexahydro-1,4-diazepine tetrahydrochloride

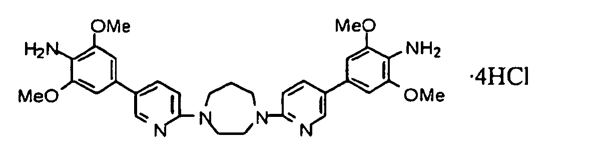

Following the procedures of Example 17, 1,4-bis[5-(4-benzyloxycarbonylamino-3,5-dimethoxyphenyl)-2-pyridyl]-hexahydro-1,4-diazepine was obtained as a colorless oil (120.0 mg, yield: 26%) from 1,4-bis(5-bromo-2-pyridyl)hexahydro-1,4-diazepine (228.0 mg, 0.550 mmol) synthesized in Referential Example 1 and 4-benzyloxycarbonylamino-1-bromo-3,5-dimethoxybenzene (450.0 mg, 1.24 mmol).

To a solution of 1,4-bis[5-(4-benzyloxycarbonylamino-3,5-dimethoxyphenyl)-2-pyridyl]hexahydro-1,4-diazepine (120.0 mg, 0.140 mmol) in acetic acid (2.0 mL), 10% palladium on charcoal (24.0 mg) was added, and under a hydrogen gas atmosphere, the resulting mixture was stirred at 50°C for 1.5 hours. Insoluble matter was filtered off by using Celite, and the filtrate was concentrated under reduced pressure. A solution of the residue in chloroform was washed with a saturated aqueous solution of sodium hydrogencarbonate and brine, dried over anhydrous sodium sulfate, and then concentrated under reduced pressure. To a solution of the residue in chloroform (5.0 mL), a 4.0 M ethyl acetate solution of hydrogen chloride (0.14 mL, 0.56 mmol) was added, and the resulting mixture wasconcentrated under reduced pressure. Ethanol (5.0 mL) was added to the residue, and the resulting mixture was concentrated under reduced pressure. Diethyl ether was added to the residue, and the resulting precipitate was collected by filtration. The title compound was obtained as pale brown amorphous powder (84.0 mg, yield: 82%).
¹H-NMR (CDCl₃) (data of the free base of the title compound; amine NH₂ protons were not observed) δ: 2.12-2.22(m, 2H), 3.59-3.66(m, 4H), 3.90(s, 12H), 3.94(s, 4H), 6.60(d, J=8.8Hz, 2H), 6.67(s, 4H), 7.64(d, J=2.0, 8.8Hz, 2H), 8.38 (d, J=2.0Hz,2H).

### Example 35

### 1,4-Bis[5-(4-dimethylamino-3,5-dimethoxyphenyl)-2-pyridyl]hexahydro-1,4-diazepine

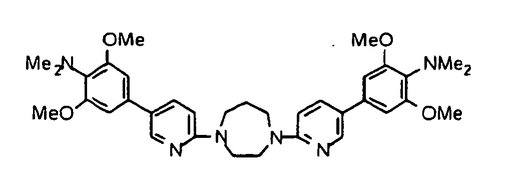

Following the procedures of Example 17, crude crystals were obtained from 1,4-bis(5-bromo-2-pyridyl)hexahydro-1,4-diazepine (210.0 mg,0.510 mmol) synthesized in Referential Example 1 and 1-bromo-4-dimethylamino-3,5-dimethoxybenzene (300.0 mg, 1.14 mmol) synthesized from 4-bromo-2,6-dimethoxyaniline [Z. Naturforsch., B24(5), 524-527(1969)],. The crude crystals were recrystallized from methanol, whereby the title compound was obtained as yellow crystalline powder (melting point: 211.0-213.0°C) (36.0 mg, yield: 12%).
¹H-NMR (CDCl₃) δ: 2.16(tt, J=6.3, 6.3Hz, 2H), 2.84(s, 12H), 3.64(dd, J=6.3, 6.3Hz, 4H), 3.89(s, 12H), 3.96(s, 4H), 6.61(d, J=9.0Hz, 2H), 6.66(s, 4H), 7.66(dd, J=2.2, 9.0Hz, 2H), 8.41(d, J=2.2Hz, 2H).

### Example 36

### 1,4-Bis[5-[3,5-dimethoxy-4-(1-pyrrolidinyl)phenyl]-2-pyridyl]hexahydro-1,4-diazepine

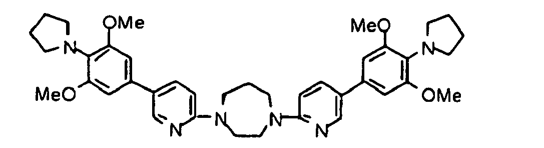

Following the procedures of Example 17, crude crystals were obtained from 1,4-bis(5-bromo-2-pyridyl)hexahydro-1,4-diazepine (280.0 mg,0.680 mmol) synthesized in Referential Example 1 and 1-bromo-3,5-dimethoxy-4-(1-pyrrolidinyl)-benzene (436.0 mg, 1.52 mmol) synthesized from 4-bromo-2,6-dimethoxyaniline [Z. Naturforsch., B24(5), 524-527 (1969)]. The crude crystals were recrystallized from methanol, whereby the title compound was obtained as pale brown crystalline powder (melting point: 204.0-207.0°C) (40.0 mg, yield: 9%).
¹H-NMR (CDCl₃) δ: 1.91-1.97(m, 8H), 2.16(tt, J=6.3, 6.3Hz, 2H), 3.26-3.33(m, 8H), 3.64(dd, J=6.3, 6.3Hz, 4H), 3.87(s, 12H), 3.95(s, 4H), 6.61(d, J=8.8Hz, 2H), 6.69(s, 4H), 7.66(dd, J=2.2, 8.8Hz, 2H), 8.41(d, J=2.2Hz, 2H).

### Example 37

### 1,4-Bis[5-(4-methanesulfonylamino-3,5-dimethoxyphenyl)-2-pyridyl]hexahydro-1,4-diazepine

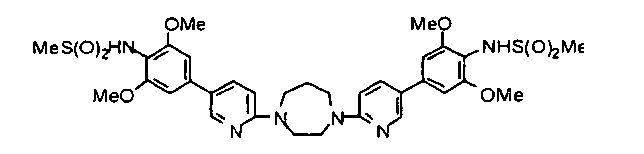

To a solution of 1,4-bis[5-(4-amino-3,5-dimethoxy-phenyl)-2-pyridyl]hexahydro-1,4-diazepine tetrahydro-chloride (40.0 mg, 0.0570 mmol), which was synthesized in Example 34, in pyridine (1.0 mL), methanesulfonyl chloride (30.0 mg, 0.260 mmol) was added. The reaction mixture was stirred for 15 minutes, and then concentrated under reduced pressure. A solution of the residue in methanol-chloroform (1:10) was washed with a saturated aqueous solution of sodium hydrogencarbonate and brine, dried over anhydrous sodium sulfate, and then concentrated under reduced pressure. The residue was recrystallized from chloroform-hexane, whereby the title compound was obtained as pale brown crystalline powder (melting point: 235.0-239.0°C) (28.0 mg, yield: 68%).
¹H-NMR (CDCl₃) δ: 2.10-2.22(m, 2H), 3.27(s, 6H), 3.60-3.70(m, 4H), 3.93(s, 12H), 3.97(s, 4H), 6.13 (s, 2H), 6.63(d, J=8.8Hz, 2H), 6.70(s, 4H), 7.65(dd, J=2.2, 8.8Hz, 2H), 8.39(d, J=2.2Hz, 2H).

### Example 38

### 1,4-Bis[5-(4-fluoro-3,5-dimethoxyphenyl)-2-pyridyl]hexahydro-1,4-diazepine

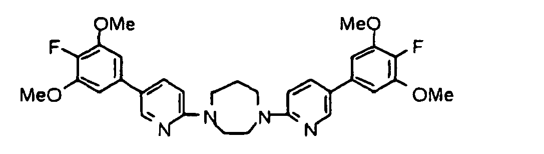

Following the procedures of Example 1, the title compound was obtained as pale yellow amorphous powder (138.9 mg, yield: 93%) from 1,4-bis(5-bromo-2-pyridyl)hexahydro-1,4-diazepine (110.0 mg, 0.270 mmol) synthesized in Referential Example 1 and 4-fluoro-3,5-dimethoxyphenylboric acid (160.0 mg, 0.800 mmol) synthesized from 1-bromo-4-fluoro-3,5-dimethoxybenzene (JP10-87543A) synthesized by similar procedures as in Referential Example 2.
¹H-NMR (CDCl₃) δ: 2.16(tt, J=6.0, 6.03Hz, 2H), 3.64(dd, J=6.0, 6.0Hz, 4H), 3.93(s, 12H), 3.95(s, 4H), 6.62 (d, J=9.0Hz, 2H), 6.71 (d, ⁴J_{Hf}=6.0Hz, 4H), 7.63(dd, J=3.0, 9.0Hz, 2H), 8.36(d, J=3.0Hz, 2H).

### Example 39

### 1,9-Bis[5-(9-chloro-3,5-dimethoxyphenyl)-2-pyridyl]hexahydro-1,4-diazepine dimethanesulfonate

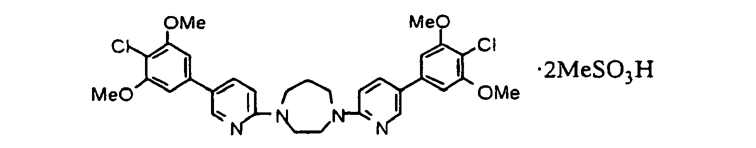

Following the procedures of Example 1, 1,4-bis[5-(4-chloro-3,5-dimethoxyphenyl)-2-pyridyl]hexahydro-1,4-diazepine was obtained as colorless crystalline powder (98.0 mg, yield: 82%) from 1,4-bis(5-bromo-2-pyridyl)hexahydro-1,4-diazepine (82.0 mg, 0.200 mmol) synthesized in Referential Example 1 and 4-chloro-3,5-dimethoxyphenylboric acid (130.0 mg, 0.600 mmol) synthesized in Referential Example 10.

To a solution of 1,4-bis[5-(4-chloro-3,5-dimethoxy-phenyl)-2-pyridyl]hexahydro-1,4-diazepine (130.0mg, 0.210mmol) in methanol (5.0 mL), a 1.0M aqueous solution of methanesulfonic acid (0.45 mL, 0. 45 mmol) was added, and the reaction mixture was concentrated under reducedpressure. Ethanol (5.0 mL) was added to the residue, and the resulting mixture was concentrated under reduced pressure. The residue was recrystallized from methanol-chloroform-diethyl ether, the title compound was obtained as colorless crystalline powder (melting point: 279.0-282.0°C) (138.0 mg, yield: 83%).
¹H-NMR (DMSO-d₆, 120°C) (ammonium salt NH⁺ protons were not observed) δ: 2.03 (tt, J=5.8, 5.8Hz, 2H), 2.46(s, 6H), 3.77(dd, J=5.8, 5.8Hz, 4H), 3.88(s, 12H), 3.99(s, 4H), 6.88(s, 4H), 6.95(d, J=9.0Hz, 2H), 7.94(dd, J=2.6, 9.0Hz, 2H), 8.35(d, J=2.6Hz, 2H).

### Example 40

### 1,4-Bis[5-(4-cyano-3,5-dimethoxyphenyl)-2-pyridyl]hexahydro-1,4-diazepine

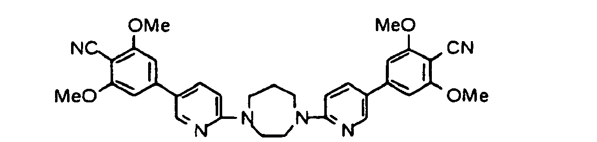

Following the procedures of Example 1, an oil was obtained from 1,4-bis(5-bromo-2-pyridyl)hexahydro-1,4-diazepine (124.0 mg, 0.300 mmol) synthesized in Referential Example 1 and 4-cyano-3,5-dimethoxyphenylboric acid (191.0 mg, 0.660 mmol) synthesized in Referential Example 11. Chloroform-hexane was added to the oil, and the resulting precipitate was collected by filtration, the title compound was obtained as pale brown crystalline powder (melting point: ≥300°C) (16.0mg, yield: 9%).
¹H-NMR (CDCl₃) δ: 2.16 (tt, J=6.1, 6.1Hz, 2H), 3.66(dd, J=6.1, 6.1Hz, 4H), 3.96(s, 12H), 3.97(s, 4H), 6.64(d, J=8.9Hz, 2H), 6.65(s, 4H), 7.68(dd, J=2.4, 8.9Hz, 2H), 8.44(d, J=2.4Hz, 2H).

### Example 41

### 1,9-Bis[5-(3,5-dimethoxy-9-methylphenyl)-2-pyridyl]hexahydro-1,4-diazepine dimethanesulfonate

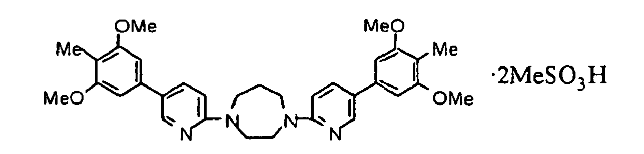

Following the procedures of Referential Example 2, 3,5-dimethoxy-4-methylphenylboric acid was synthesized from 1-iodo-3,5-dimethoxy-4-methylbenzene which had been synthesized as an intermediate in Referential Example 11.

Following the procedures of Example 1, 1,4-bis[5-(3,5-dimethoxy-4-methylphenyl)-2-pyridyl]hexahydro-1,4-diazepine was obtained as a pale yellow oil (77. 0 mg, yield: 58%) from 1,4-bis(5-bromo-2-pyridyl)hexahydro-1,4-diazepine (100.0 mg, 0.240 mmol) synthesized in Referential Example 1 and 3,5-dimethoxy-4-methylhenylboric acid (103.0 mg, 0.530 mmol)

To a solution of 1,4-bis[5-(3,5-dimethoxy-4-methyl-phenyl)-2-pyridyl]hexahydro-1,4-diazepine (77.0 mg, 0.140 mmol) in methanol (5.0 mL), a 1.0 M aqueous solution of methanesulfonic acid (0.28 mL, 0.28 mmol) was added, and the reaction mixture was concentrated under reduced pressure. Ethanol (5.0 mL) was added to the residue, and the resulting mixture was concentrated under reduced pressure. The residue was recrystallized from methanol-chloroform-diethyl ether, the title compound was obtained as pale yellow crystalline powder (melting point: 235.0-237.0°C (67.0 mg, yield: 64%).
¹H-NMR (CDCl₃) (data of the free base of the title compound) δ: 2.12(s, 6H), 2.17(tt, J=6.0, 6.0Hz, 2H), 3.64(dd, J=6.0, 6.0Hz, 4H), 3.87(s, 12H), 3.96(s, 4H), 6.62(d, J=9.0Hz, 2H), 6.66(s, 4H), 7.68(dd, J=2.2, 9.0Hz, 2H), 8.42(d, J=2.2Hz, 2H).

### Example 42

### 1,4-Bis[5-(4-hydroxymethyl-3,5-dimethoxyphenyl)-2-pyridyl]hexahydro-1,4-diazepine

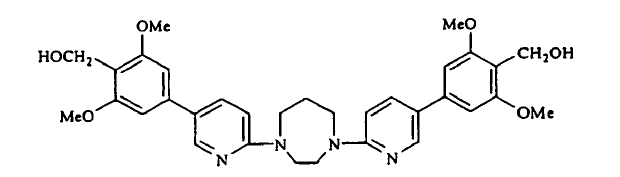

Following the procedures of Example 17, an oil (192.0 mg) containing 1,4-bis[5-[4-(t-butyldimethylsiloxy)methyl-3,5-dimethoxyphenyl]-2-pyridyl]hexahydro-1,4-diazepine was obtained from 1,4-bis(5-bromo-2-pyridyl)hexahydro-1,4-diazepine (128.0 mg, 0.310 mmol) synthesized in Referential Example 1 and 4-(5-butyldimethylsiloxy)methyl-3,5-dimethoxyphenyl trifluoromethanesulfonate (400.0 mg, 0.929 mmol) synthesized in Referential Example 12,

To a solution of the oil (192.0mg), which had been obtained by the above procedures, in methanol (9.5 mL), 46% hydrofluoric acid (0.50 mL) was added, and the resulting mixture was stirred at room temperature for 30 minutes. A 5.0 M aqueous solution of sodium hydroxide (5.0 mL) was added to the reaction mixture. The thus-obtained mixture was extracted with chloroform, and the organic layer was concentrated under reduced pressure. The residue was purified by chromatography on a silica gel column, whereby an oil was obtained. The oil was recrystallized from methylene chloride-diethyl ether-hexane, whereby the title compound was obtained as slightly yellow crystalline powder (melting point: ≥300.0°C) (29.0 mg, yield: 16% based on the 1,4-bis(5-bromo-2-pyridyl)-hexahydro-1,4-diazepine).
¹H-NMR (CDCl₃) δ: 2.14-2.28 (m, 2H), 2.41(br.t, J=5.9Hz, 2H), 3.89(s, 12H), 3.55-4.15(m, 8H), 4.80(br.d, J=5.9Hz, 4H), 6.64(br.d, J=8.8Hz, 2H), 6.67(s, 4H), 7.69(br.d, J=8.8Hz, 2H), 8.42(br.s, 2H).

### Example 43

### 1,4-Bis[5-(4-ethoxycarbonyl-3,5-dimethoxyphenyl)-2-pyridyl]hexahydro-1,4-diazepine

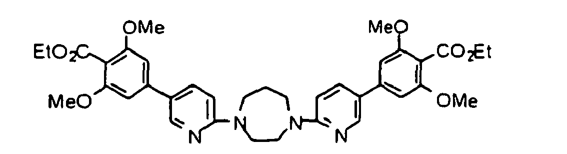

Following the procedures of Example 17, an oil was obtained from 1,4-bis(5-bromo-2-pyridyl)hexahydro-1,4-diazepine (243.0 mg, 0.590 mmol) synthesized in Referential Example 1 and 4-ethoxycarbonyl-1-iodo-3,5-dimethoxybenzene (595.0 mg, 0.929 mmol) synthesized in Referential Example 13. The oil was recrystallized from methylene chloride-diethyl ether-hexane, whereby the title compound was obtained as pale brown crystalline powder (melting point: 244.0-245.0°C) (75.1 mg, yield: 19%).
¹H-NMR (CDCl₃) δ: 1.38(br.t, J=7.0Hz, 6H), 2.12-2.25(m, 2H), 3.87(s, 12H), 3.60-4.05(m, 8H), 4.40(br.q, J=7.0Hz, 4H), 6.62(br.d, J=8.8Hz, 2H), 6.66(s, 4H), 7.66(br.dd, J=2.4, 8.8Hz, 2H), 8.40(br.d, J=2.4Hz, 2H).

### Example 44

### 1,4-Bis[5-(4-carboxy-3,5-dimethoxyphenyl)-2-pyridyl]-hexahydro-1,4-diazepine ditrifluoroacetate

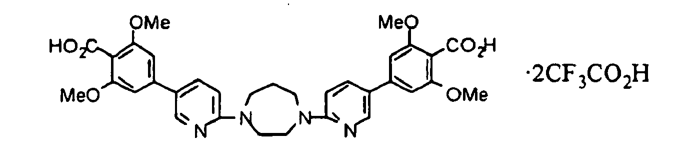

Following the procedures of Example 1, 1,4-bis[5-(4-t-butoxycarbonyl-3,5-dimethoxyphenyl)-2-pyridyl]hexahydro-1,4-diazepine was obtained as a pale yellow oil(27.0 mg, yield: 25%) from 1,4-bis(5-bromo-2-pyridyl)hexahydro-1,4-diazepine (62.0 mg, 0.150 mmol) synthesized in Referential Example 1 and 4-t-butoxycarbonyl-3,5-dimethoxyphenylboric acid (102.0 mg, 0.360 mmol) synthesized in Referential Example 14.

To a solution of 1,4-bis[5-(4-t-butoxycarbonyl-3,5-dimethoxyphenyl)-2-pyridyl]hexahydro-1,4-diazepine (27.0 mg, 0.037 mmol) in chloroform (4.0mL), trifluroroacetic acid (0.3 mL) was added, and the resulting mixture was stirred for 30 minutes. The reaction mixture was concentrated under reduced pressure. Methanol-chloroform-diethyl ether-hexane was added to the residue and the resulting precipitate was collected, whereby the title compound was obtained as pale yellow crystalline powder [melting point: 164.5°C (decomposed)](22.6 mg, yield: 72%).
¹H-NMR [CD₃OD-CDCl₃(1:10)] (neither ammonium salt NH⁺ protons nor carboxylic acid CO₂H protons were observed) δ: 2.12-2.28(m, 2H), 3.89(br.s, 12H), 3.70-4.07(m, 8H), 6.69(br.s, 4H), 6.82(br.d, J=9.8Hz, 2H), 7.82(br.d, J=9.8Hz, 2H), 8.34(br.s, 2H).

### Example 45

### 1,4-Bis[5-(4-acetyl-3,5-dimethoxyphenyl)-2-pyridyl]-hexahydro-1,4-diazepine dimethanesulfonate

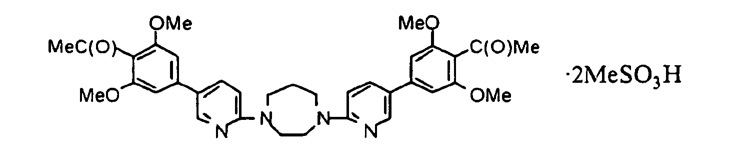

Following the procedures of Example 17, 1,4-bis[5-(4-acetyl-3,5-dimethoxy-phenyl)-2-pyridyl]hexahydro-1,4-diazepine (162.0 mg) was obtained as colorless crystalline powder (yield: 53%) from 1,4-bis(5-bromo-2-pyridyl)-hexahydro-1,4-diazepine (206.0 mg, 0.500 mmol) synthesized in Referential Example 1 and 4-acetyl-3,5-dimethoxyphenyl trifluoro-methanesulfonate (410.0 mg, 1.250 mmol) synthesized in Referential Example 15.

To a solution of 1,4-bis[5-(4-acetyl-3,5-dimethoxy-phenyl)-2-pyridyl]hexahydro-1,4-diazepine (80.0mg, 0.130mmol) inchloroform (5.0 mL), a 1.0 M methanol solution of methanesulfonic acid (0.33 mL, 0.33 mmol) was added, and the reaction mixture was concentrated under reduced pressure. The residue was recrystallized from methanol-diethyl ether, whereby the title compound was obtained as colorless crystalline powder (melting point: 236.0-238.0°C)(94.8 mg, yield: 90%).
¹H-NMR (CDCl₃) (ammonium salt NH⁺ protons were not observed) δ: 2.34-2.42(m, 2H), 2.48(s, 6H), 2.91(s, 6H), 3.84(s, 12H), 3.98-4.07(m, 4H), 4.31(s, 4H), 6.61(s, 4H), 7.41(br.d, J=9.4Hz, 2H), 8.15(br.d, J=9.4Hz, 2H), 8.43 (br.s, 2H).

### Example 46

### 1,4-Bis[5-(4-methoxy-3,5-dimethylphenyl)-2-pyridyl]-hexahydro-1,4-diazepine dimethanesulfonate

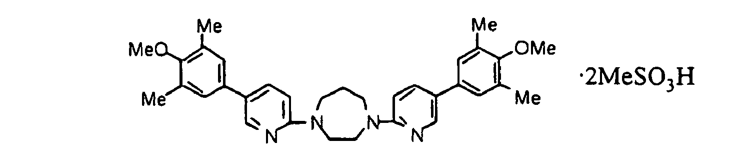

Following the procedures of Example 1, 1,4-bis[5-(4-methoxy-3,5-dimethylphenyl)-2-pyridyl]hexahydro-1,4-diazepine was obtained as colorless crystalline powder (130.0 mg, yield: 82%) from 1,4-bis(5-bromo-2-pyridyl)hexahydro-1,4-diazepine (123.0mg, 0.300 mmol) synthesized in Referential Example 1 and 4-methoxy-3,5-dimethylphenylboric acid (152.0 mg, 0.900 mmol) synthesized from 1-bromo-4-methoxy-3,5-dimethylbenzene [Tetrahedron, Lett., 30, 735-738 (1989)] by similar procedures as in Referential Example 2.

To a solution of 1,4-bis[5-(4-methoxy-3,5-dimethyl-phenyl)-2-pyridyl]hexahydro-1,4-diazepine (126.0 mg, 0.240 mmol) in methanol (10 mL), a 1.0 M aqueous solution of methanesulfonic acid (0.50 mL, 0.50 mmol) was added, and the reaction mixture was then concentrated under reduced pressure. Ethanol (10 mL) was added to the concentration residue, and the resulting mixture was concentrated under reduced pressure. The residue was recrystallized from ethanol-diethyl ether-hexane, whereby the title compound was obtained as colorless crystalline powder (melting point: 250.0-253.0°C) (157.0 mg, yield: 91%).
¹H-NMR (DMSO-d₆, 120°C) (ammonium salt NH⁺ protons were not observed) δ: 2.01(tt, J=5.8, 5.8Hz, 2H), 2.24(s, 12H), 2.45(s, 6H), 3.68(s, 6H), 3.77(dd, J=5.8, 5.8Hz, 4H), 4.00(s, 4H), 6.98(d, J=9.0Hz, 2H), 7.14(s, 4H), 7.86(dd, J=2.4, 9.0Hz, 2H), 8.15(d, J=2.4Hz, 2H).

### Example 47

### 1,4-Bis[5-(3-formyl-2-furyl)-2-pyridyl]hexahydro-1,4-diazepine

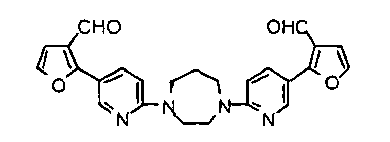

Following the procedures of Example 1, the title compound was obtained as brown amorphous powder (8.0 mg, yield: 9%) from 1,4-bis(5-bromo-2-pyridyl)hexahydro-1,4-diazepine (82.0 mg, 0.200 mmol) synthesized in Referential Example 1 and 3-formylfuran-2-boric acid (62.0 mg, 0.440 mmol).
¹H-NMR (CDCl₃) δ: 2.15(tt, J=6.0, 6.0Hz, 2H), 3.68(dd, J=6.0, 6.0Hz, 4H), 3.99(s, 4H), 6.65(d, J=9.0Hz, 2H), 6.87(d, J=2.1Hz, 2H), 7.42(d, J=2.1Hz, 2H), 7.87(dd, J=2.4, 9.0Hz, 2H), 8.57(d, J=2.4Hz, 2H), 10.04(s, 2H).

### Example 48

### 1,4-Bis[5-(3-thienyl)-2-pyridyl]hexahydro-1,4-diazepine

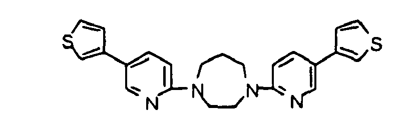

Following the procedures of Example 1, crude crystals were obtained from 1,4-bis(5-bromo-2-pyridyl)hexahydro-1,4-diazepine (82.0 mg,0.200 mmol) synthesized in Referential Example 1 and thiophene-3-boric acid (56.0 mg, 0.44 mmol). Methanol-chloroform-diethyl ether was added to the crude crystals and the resulting precipitate was collected by filtration, whereby the title compound was obtained as brown needles (melting point: 234.0-236.0°C) (35.0mg, yield: 42%).
¹H-NMR (CDCl₃) δ: 2.14(tt, J=6.3, 6.3Hz, 2H), 3.61(dd, J=6.3, 6.3Hz, 4H), 3.93(s, 4H), 6.58(d, J=9.0Hz, 2H), 7.28-7.33(m, 4H), 7.36-7.39(m, 2H), 7.66(dd, J=2.4, 9.0Hz, 2H), 8.44(d, J=2.4Hz, 2H).

### Example 49

### 1,4-Bis[5-(2-formyl-3-thienyl)-2-pyridyl]hexahydro-1,4-diazepine

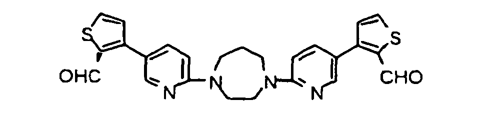

Following the procedures of Example 1, the title compound was obtained as pale yellow amorphous powder (71.0 mg, yield: 74%) from 1,4-bis(5-bromo-2-pyridyl)hexahydro-1,4-diazepine (82.0 mg, 0.200 mmol) synthesized in Referential Example 1 and 2-formylthiophene-3-boric acid (69.0 mg, 0.44 mmol).
¹H-NMR (DMSO-d₆,120°C) δ: 2.02(tt, J=6.0, 6.0Hz, 2H), 3.71(dd, J=6.0, 6.0Hz, 4H), 3.94(s, 4H), 6.77(d, J=8.7Hz, 2H), 7.27(d, J=4.8Hz, 2H), 7.65(dd, J=2.6, 8.7Hz, 2H), 7.97(dd, J=1.2, 4.8Hz, 2H), 8.25(d, J=2.6Hz, 2H), 9.78(d, J=1.2Hz, 2H).

### Example 50

### 1,4-Bis[5-(2-thienyl)-2-pyridyl]hexahydro-1,4-diazepine

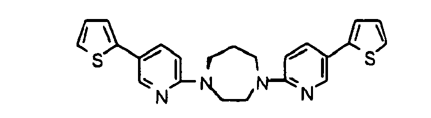

Following the procedures of Example 1, crude crystals were obtained from 1,4-bis(5-bromo-2-pyridyl)hexahydro-1,4-diazepine (82.0 mg, 0.200 mmol) synthesized in Referential Example 1 and thiophene-2-boric acid (56.0 mg, 0.44 mmol). Methanol-chloroform-diethyl ether was added to the crude crystals and the resulting precipitate was collected by filtration, whereby the title compound was obtained as brown crystalline powder (melting point: 170.0-172.0°C) (21.0 mg, yield: 25%).
¹H-NMR (DMSO-d₆,120°C) δ: 1.99(tt, J=6.0, 6.0Hz, 2H), 3.65(dd, J=6.0, 6.0Hz, 4H), 3.88(s, 4H), 6.91(d, J=9.0Hz, 2H), 7.03(dd, J=3.4, 5.1Hz, 2H), 7.19(dd, J=1.2, 3.4Hz, 2H), 7.30(dd, J=1.2, 5.1Hz, 2H), 7.65(dd, J=2.6, 9.0Hz, 2H), 8.31(d, J=2.6Hz, 2H).

### Example 51

### 1,4-Bis[5-(5-acetyl-2-thienyl)-2-pyridyl]hexahydro-1,4-diazepine

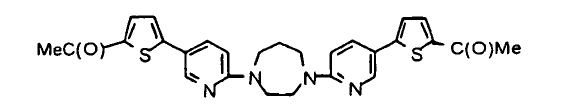

Following the procedures of Example 1, the title compound was obtained as yellow amorphous powder (12.0 mg, yield: 11%) from 1,4-bis(5-bromo-2-pyridyl)hexahydro-1,4-diazepine (82.0 mg, 0.200 mmol) synthesized in Referential Example 1 and 5-acetylthiophene-2-boric acid (75.0 mg, 0.44 mmol).
¹H-NMR (DMSO-d₆,120°C) δ: 1.99(tt, J=6.0, 6.0Hz, 2H), 2.46(s, 6H), 3.67(dd, J=6.0, 6.0Hz, 4H), 3.91(s, 4H), 6.72 (d, J=9.0 Hz, 2H), 7.29 (d, J=4.1Hz, 2H), 7.72(dd, J=2.4, 9.0Hz, 2H), 7.73(d, J=4.1Hz, 2H), 8.42(d, J=2.4Hz, 2H).

### Example 52

### 1,4-Bis[5-(3-pyridyl)-2-pyridyl]hexahydro-1,4-diazepine

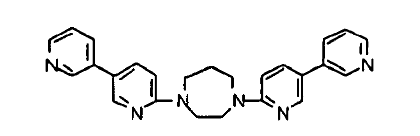

Following the procedures of Example 1, crude crystals were obtained from 1,4-bis(5-bromo-2-pyridyl)hexahydro-1,4-diazepine (82.0 mg,0.200 mmol) synthesized in Referential Example 1 and pyridine-3-boric acid (54.0 mg, 0.44 mmol). The crude crystals were recrystallized from chloroform-hexane, whereby the title compound was obtained as colorless scales (melting point: 210.0-211.0°C) (38.0 mg, yield: 45%).
¹H-NMR (CDCl₃) δ: 2.17(tt, J=6.0, 6.0Hz, 2H), 3.65(dd, J=6.0, 6.0Hz, 4H), 3.97(s, 4H), 6.66(d, J=9.0Hz, 2H), 7.33(dd, J=4.8, 7.8Hz, 2H), 7.68(dd, J=2.4, 9.0Hz, 2H), 7.80(ddd, J-1.7, 2.1, 7.8Hz, 2H), 8.41(d, J=2.4Hz, 2H), 8.53(dd, J=1.7, 4.8Hz, 2H), 8.78(d, J=2.1Hz, 2H).

### Example 53

### 1,4-Bis[5-(2-benzo[b]furyl)-2-pyridyl]hexahydro-1,4-diazepine

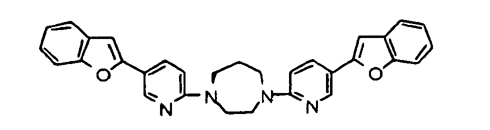

Following the procedures of Example 1, crude crystals were obtained from 1,4-bis(5-bromo-2-pyridyl)hexahydro-1,4-diazepine (82.0 mg,0.200 mmol) synthesized in Referential Example 1 and benzo[b]furan-2-boric acid (71.0 mg, 0.44 mmol). Methanol-chloroform-diethyl ether was added to the crude crystals and the resulting precipitate was collected by filtration, whereby the title compound was obtained as colorless crystalline powder [melting point: 245.0°C (decomposed)](17.0 mg, yield: 17%).
¹H-NMR (DMSO-d₆, 120°C) δ: 2.02 (tt, J=6.0, 6.0Hz, 2H), 3.70(dd, J=6.0, 6.0Hz, 4H), 3.94(s, 4H), 6.77(d, J=9.0Hz, 2H), 6.97-6.99(m, 2H), 7.14-7.23(m, 4H), 7.44-7.56(m, 4H), 7.87(dd, J=2.4, 9.0Hz, 2H), 8.57 (d, J=2. 4Hz, 2H).

### Example 54

### 1,4-Bis[5-(2-benzo[b]thienyl)-2-pyridyl]hexahydro-1,4-diazepine

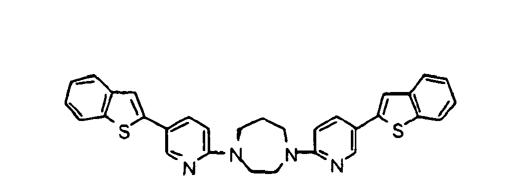

Following the procedures of Example 1, crude crystals were obtained from 1,4-bis(5-bromo-2-pyridyl)hexahydro-1,4-diazepine (82.0 mg,0.200 mmol) synthesized in Referential Example 1 and benzo[b]thiophene-2-boric acid (78.0 mg, 0.44 mmol). Methanol-chloroform-diethyl ether was added to the crude crystals and the resulting precipitate was collected by filtration, whereby the title compoundwas obtained as colorless crystalline powder [melting point: 246.0°C (decomposed)] (41.0 mg, yield: 39%).
¹H-NMR (DMSO-d₆, 120°C) δ: 2.01(tt, J=6.0, 6.0Hz, 2H), 3.69(dd, J=6.0, 6.0Hz, 4H), 3.92(s, 4H), 6.75(d, J=9.0Hz, 2H), 7.20-7.35(m, 4H), 7.49(s, 2H), 7.67-7.75(m, 2H), 7.77(dd, J=2.4,9.0Hz, 2H), 7.80-7.84(m, 2H), 8.43 (d, J=2.4Hz, 2H).

### Example 55

### 1,9-Bis[5-(3,9,5-trimethoxyphenyl)-2-pyridyl]-piperazine dimethanesulfonate

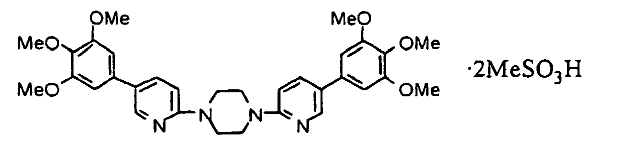

Following the procedures of Example 1, 1,4-bis[5-(3,4,5-trimethoxyphenyl)-2-pyridyl]-piperazine was obtained as colorless crystalline powder (147.0 mg, yield: 52%) from 1,4-bis(5-bromo-2-pyridyl)piperazine (199.0mg, 0.500 mmol) synthesized in a similar manner as in Referential Example 1 and 3,4,5-trimethoxyphenylboric acid (318.0 mg, 1.50 mmol).

To a solution of 1,4-bis[5-(3,4,5-trimethoxyphenyl)-2-pyridyl]piperazine (147.0 mg, 0.257 mmol) in methylene chloride (5.0 mL), a 1.0 M methanol solution of methanesulfonic acid (0.54 mL, 0.54 mmol) was added, and the reaction mixture was then concentrated under reduced pressure. The residue was recrystallized from methylene chloride-diethyl ether, whereby the title compound was obtained as pale yellow crystalline powder (melting point: 238.0-239.0°C) (160.8 mg, yield: 82%).
¹H-NMR (CD₃OD) (ammonium salt NH⁺ protons were not observed) δ: 2.72(s, 6H), 3.82(s, 6H), 3.93(s, 12H), 4.09(s, 8H), 6.89(s, 4H), 7.44(d, J=9.5Hz, 2H), 8.24(s, 2H), 8.38(d, J=9.5Hz, 2H).

### Example 56

### 4,8-Bis[5-(3,4,5-trimethoxyphenyl)-2-pyridyl]-1,4,8-triazacyclo[4.4.0]decane

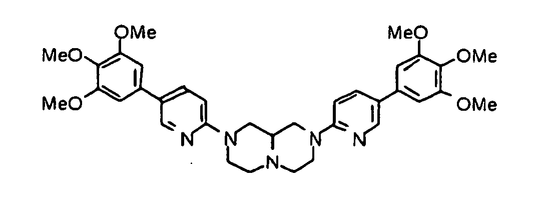

Following the procedures of Example 1, the title compound was obtained as pale brown amorphous powder (89.9 mg, yield: 71%) from 4,8-bis(5-bromo-2-pyridyl)-1,4,8-triazacyclo-[4.4.0]decane (91.7 mg, 0.202 mmol) synthesized in a similar manner as in Referential Example 1 and 3,4,5-trimethoxy-phenylboric acid (133.3 mg, 0.628 mmol).
¹H-NMR (CDCl₃) δ: 2.37(br.tt, J=2.3, 12.0Hz, 1H), 2.43(ddd, J=3.2, 12.0, 12.0Hz, 2H), 2.78(dd, J=12.0, 12.0Hz, 2H), 2.99(br.d, J=12.0Hz, 2H), 3.15(ddd, J=2.7, 12.0, 12.0Hz, 2H), 3.88(s, 6H), 3.92(s, 12H), 4.25(br.d, J=12.0Hz, 2H), 4.33(br.d, J=12.0Hz, 2H), 6.70(s, 4H), 6.74(d, J=8.8Hz, 2H), 7.70(dd, J=2.5,8.8Hz, 2H), 8.42(d, J=2.5Hz, 2H).

### Example 57

### 1,3-Bis[4-[5-(3,4,5-trimethoxyphenyl)-2-pyridyl]-1-piperazinyl]propane

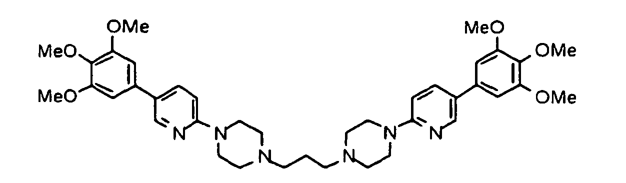

Following the procedures of Example 1, an oil was obtained from 1,3-bis[4-(5-bromo-2-pyridyl)-1-piperazinyl]-propane (196.0 mg, 0.370 mmol) synthesized in a similar manner as in Referential Example 1 and 3,4,5-trimethoxyphenylboric acid (244.0 mg, 1.32 mmol). The oil was recrystallized from chloroform-hexane, whereby the title compound was obtained as colorless crystalline powder (melting point: 175.0-176.0°C) (78.4 mg, yield: 34%).
¹H-NMR (CDCl₃) δ: 1.82 (br.quint, J=7.4Hz, 2H), 2.48(br.t, J=7.4Hz, 4H), 2.61(br.dd, J=4.7, 4.7Hz, 8H), 3.62(br.dd, J=4.7, 4.7Hz, 8H), 3.88(s, 6H), 3.91(s, 12H), 6.70(s, 4H), 6.72(d, J=8.9Hz, 2H), 7.68(dd, J=2.5, 8.9Hz, 2H), 8.41(d, J=2.5Hz, 2H).

### Example 58

### N,N'-Bis[5-(3,4,5-trimethoxyphenyl)-2-pyridyl]-N,N'-dimethylethylenediamine dimethanesulfonate

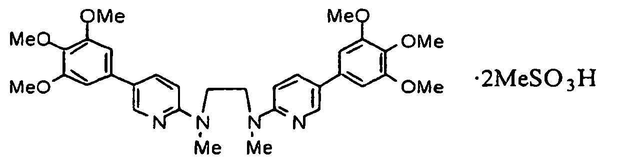

Following the procedures of Example 1, N,N'-bis[5-(3,4,5-trimethoxyphenyl)-2-pyridyl]-N,N'-dimethylethylene-diamine was obtained as a pale yellow oil (40.0 mg, yield: 56%) from N,N'-bis(5-bromo-2-pyridyl)-N,N'-dimethylethylene-diamine (50.0 mg, 0.120 mmol) synthesized in a similar manner as in Referential Example 1 and 3,4,5-trimethoxy-phenylboric acid (79.5 mg, 0.375 mmol).

To a solution of N,N'-bis[5-(3,4,5-trimethoxyphenyl)-2-pyridyl]-N,N'-dimethylethylenediamine (40.0 mg, 0.070 mmol) in methanol (5.0 mL), a 1.0 M aqueous solution of methanesulfonic acid (0.14 mL, 0.14 mmol) was added, and the reaction mixture was concentrated under reduced pressure. Ethanol (5.0 mL) was added to the residue, and the mixture was concentrated under reduced pressure. The residue was recrystallized from methanol-diethylether, whereby the title compound was obtained as pale yellow crystalline powder (melting point: 204.0-206.0°C) (32.0 mg, yield: 60%).
¹H-NMR (DMSO-d₆, 120°C) (ammonium salt NH⁺ protons were not observed) δ: 2.41(s, 6H), 3.15(s, 6H), 3.73(s, 6H), 3.84(s, 12H), 3.88(s, 4H), 6.83(s, 4H), 6.91(d, J=9.0Hz, 2H), 7.93(dd, J=2.4, 9.0Hz, 2H), 8.30(d, J=2.4Hz, 2H).

### Test 1 (Evaluation of IgE antibody production inhibiting activity)

From a mouse (Balb/C, male, 8 weeks old), the spleen was excised. The spleen was shredded in 0.3% BSA/HBSS, and was then disintegrated into single cells through a 70-mesh screen. Those single cells were hemolyzed with the Gey's solution, and using RPMI 1640 medium/25 M HEPES/0.3% BSA, a spleen cell suspension (1 x 10⁷ cells/mL) was prepared. After an aliquot of the suspension was reacted with a rat anti-mouse Thy-1,2 monoclonal antibody (product of Cedarlane Laboratories Limited) at 4°C for 1 hour, centrifugation was conducted. Precipitated cells were suspended again (1 x 10⁷ cells/mL, RPMI/HEPES/BSA). After the suspension was next reacted with a low cytotoxic rabbit complement (product of Cedarlane Laboratories Limited) at 37°C for 1 hour, dead cells were removed by specific gravity centrifugation so that a B cell fraction was obtained as viable cells.

Using a 96-well plate, the B cells (10⁵ cells/0.2 mL/well) was incubated together with LPS (E. coli 026:B6, product of DIFCO Laboratories, Inc.) for 1 day. Mouse IL-4 (product of Genzyme Corp.) was then added, followed by further incubation for 6 days.

The IgE antibody production inhibiting activity of each drug was calculated by adding the drug Day 1 of the incubation and assaying the quantity of IgE in the culture supernatant after the incubation. Inhibition activity (IC₅₀) is presented in Table 1.

Further, the water solubility (%) of each compound was also determined. The results are presented in Table 1.

**Table 1**

| Compound- | IC₅₀ (µM) | Water solubility |
|---|---|---|
| (Example No.) | | (%) |
| 26 | 0.2 | 5 |
| 34 | 0.3 | 5 |
| 35 | 0.2 | 5* |
| 57 | 0.1 | 5* |
| 58 | 0.2 | 5 |

| | | |
|---|---|---|
| * Solubility in 0.1 M hydrochloric acid | | |

### Industrial Applicability

The bis(5-aryl-2-pyridyl) derivatives (1) according to the present invention and salts thereof have excellent IgE antibody production inhibiting activity and are useful as medicines for the prevention or treatment of allergic immune diseases.

## Claims

1. A bis(5-aryl-2-pyridyl) derivative represented by the following formula (1) or a salt thereof: 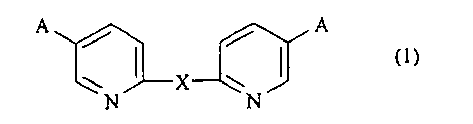 wherein A represents a substituted or unsubstituted aromatic hydrocarbon group or a substituted or unsubstituted aromatic heterocyclic group, and X represents a group selected from the following formulas (2) to (5): 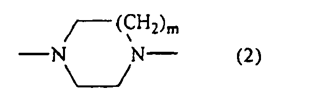 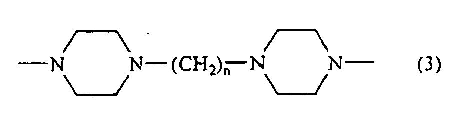 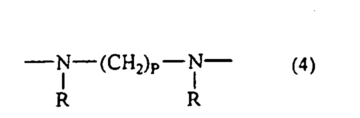 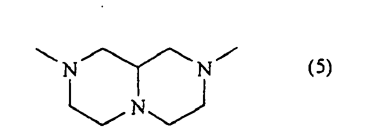 wherein in the formula (2), m stands for an integer of 1 or 2; in the formula (3), n stands for an integer of 1 to 6; in the formula (4), R represents a hydrogen atom or a lower alkyl group and p stands for an integer of 1 to 6, and in which the lower alkyl moiety is a linear, branched or cyclic alkyl group having 1 to 6 carbon atoms.

2. A bis(5-aryl-2-pyridyl) derivative or a salt thereof according to claim 1, wherein in the formula (1), A is a substituted or unsubstituted phenyl group, a substituted or unsubstituted pyridyl group, a substituted or unsubstituted thienyl group, a substituted or unsubstituted furyl group, a substituted or unsubstituted benzofuryl group, or a substituted or unsubstituted benzothienyl group.

3. A bis(5-aryl-2-pyridyl) derivative or a salt thereof according to claim 1, wherein in the formula (1), A is a substituted or unsubstituted phenyl group.

4. A bis(5-aryl-2-pyridyl) derivative or a salt thereof according to claim 1, wherein said aromatic hydrocarbon group or said aromatic heterocyclic group is substituted by 1 to 3 substituents selected from lower alkyl groups, halogeno(lower alkyl) groups, hydroxy(lower alkyl) groups, lower alkoxy(lower alkyl) groups, lower alkoxy groups, halogen atoms, hydroxy groups, cyano groups, (lower alkyl)thio groups, amino groups, mono- or di-(lower alkyl)amino groups, (lower alkyl)-sulfonylamino groups, formyl groups, carboxyl groups, (lower alkoxy)carbonyl groups, lower alkanoyl groups, pyrrolidinyl groups, and alkylenedioxy groups; in which the lower alkyl moieties are linear, branched or cyclic alkyl groups having 1 to 6 carbon atoms.

5. A bis(5-aryl-2-pyridyl) derivative or a salt thereof according to claim 1, wherein in the formula (1), X is the group represented by the formula (2).

6. A medicine comprising as an active ingredient a bis(5-aryl-2-pyridyl) derivative or a salt thereof according to any one of claims 1-5.

7. A medicine according to claim 6, which is a preventive or therapeutic for an allergic immune disease.

8. A medicine according to claim 6, which is a preventive or therapeutic for asthma, atopic dermatitis, allergic rhinilis, inflammatory colitis, contact skin disease or an allergic eye disease.

9. Use of a bis(5-aryl-2-pyridyl)derivative or a salt thereof according to any one of claims 1-5 for the manufacture of a medicine for treating or preventing an allergic immune disease.

10. The use according to claim 9, wherein the medicine is a preventive or therapeutic for asthma, atopic dermatitis, allergic rhinitis, inflammatory colitis, contact skin diseases or allergic eye diseases.

11. An IgE antibody production inhibitor which is a bis(5-aryl-2-pyridyl) derivative or a salt thereof according to any one of claims 1-5.

12. A medicinal composition comprising a bis(5-aryl-2-pyridyl) derivative or a salt thereof according to any one of claims 1-5 and a pharmacologically acceptable carrier.

## Patentansprüche

1. Bis(5-aryl-2-pyridyl)-Derivat der folgenden Formel (1) oder ein Salz davon: 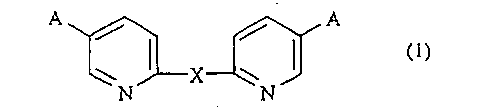 worin A eine substituierte oder unsubstituierte aromatische Kohlenwasserstoffgruppe oder eine substituierte oder unsubstituierte aromatische heterocyclische Gruppe bedeutet, und X eine Gruppe bedeutet, ausgewählt aus den folgenden Formeln (2) bis (5): 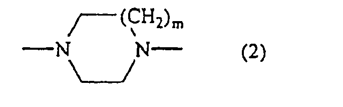 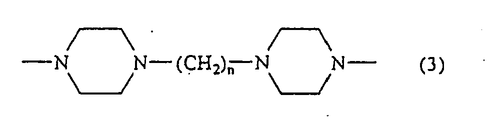 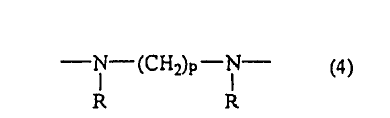 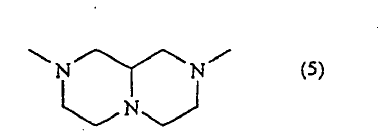 worin in Formel (2) m für eine ganze Zahl von 1 oder 2 steht; in Formel (3) n für eine ganze Zahl von 1 bis 6 steht; in Formel (4) R ein Wasserstoffatom oder eine Niederalkylgruppe bedeutet und p für eine ganze Zahl von 1 bis 6 steht, und worin die Niederalkylgruppe eine lineare, verzweigte oder cyclische Alkylgruppe mit 1 bis 6 Kohlenstoff atomen ist.

2. Bis(5-aryl-2-pyridyl)-Derivat oder ein Salz davon nach Anspruch 1, worin in Formel (1) A eine substituierte oder unsubstituierte Phenylgruppe, eine substituierte oder unsubstituierte Pyridylgruppe, eine substituierte oder unsubstituierte Thienylgruppe, eine substituierte oder unsubstituierte Furylgruppe, eine substituierte oder unsubstituierte Benzofurylgruppe oder eine substituierte oder unsubstituierte Benzthienylgruppe ist.

3. Bis(5-aryl-2-pyridyl)-Derivat oder ein Salz davon nach Anspruch 1, worin in Formel (1) A eine substituierte oder unsubstituierte Phenylgruppe ist.

4. Bis(5-aryl-2-pyridyl)-Derivat oder ein Salz davon nach Anspruch 1, worin die aromatische Kohlenwasserstoffgruppe oder die aromatische heterocyclische Gruppe durch 1 bis 3 Substituenten substituiert ist, ausgewählt aus Niederalkylgruppen, Halogen(niederalkyl)-gruppen, Hydroxy(niederalkyl)gruppen, Niederalkoxy(niederalkyl)gruppen, Niederalkoxygruppen, Halogenatomen, Hydroxygruppen, Cyangruppen, (Niederalkyl)thiogruppen, Aminogruppen, Mono- oder Di(niederalkyl)aminogruppen, (Niederalkyl)sulfonylaminogruppen, Formylgruppen, Carboxylgruppen, (Niederalkoxy)carbonylgruppen, Niederalkanoylgruppen, Pyrrolidinylgruppen und Alkylendioxygruppen; worin die Niederalkylgruppen lineare, verzweigte oder cyclische Alkylgruppen mit 1 bis 6 Kohlenstoffatomen sind.

5. Bis(5-aryl-2-pyridyl)-Derivat oder ein Salz davon nach Anspruch 1, worin in der Formel (1) X die durch Formel (2) repräsentierte Gruppe ist.

6. Arzneimittel, das als aktiven Bestandteil ein Bis(5-aryl-2-pyridyl)-Derivat oder ein Salz davon nach einem der Ansprüche 1 bis 5 aufweist.

7. Arzneimittel nach Anspruch 6, das ein Prophylaktikum oder Therapeutikum für eine allergische Immunerkrankung ist.

8. Arzneimittel nach Anspruch 6, das ein Prophylaktikum oder Therapeutikum für Asthma, atopische Dermatitis, allergische Rhinitis, entzündliche Colitis, Kontakt-Dermatose oder eine allergische Augenerkrankung ist.

9. Verwendung eines Bis(5-aryl-2-pyridyl)-Derivats oder eines Salzes davon nach einem der Ansprüche 1 bis 5 zur Herstellung eines Arzneimittels zur Behandlung oder Vorbeugung einer allergischen Immunerkrankung.

10. Verwendung nach Anspruch 9, worin das Arzneimittel ein Prophylaktikum oder Therapeutikum für Asthma, atopische Dermatitis, allergische Rhinitis, entzündliche Colitis, Kontakt-Dermatosen oder allergische Augenerkrankungen ist.

11. IgE-Antikörper-Produktionsinhibitor, der ein Bis(5-aryl-2-pyridyl)-Derivat oder ein Salz davon nach einem der Ansprüche 1 bis 5 ist.

12. Arzneimittelzusammensetzung, die ein Bis(5-aryl-2-pyridyl)-Derivat oder ein Salz davon nach einem der Ansprüche 1 bis 5 und einen pharmakologisch annehmbaren Träger umfasst.

## Revendications

1. Dérivé bis(5-aryl-2-pyridyle) représenté par la formule (1) suivante ou sel de celui-ci : 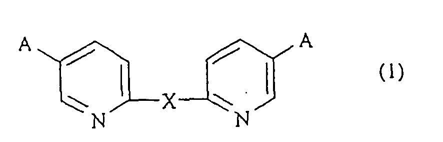 dans laquelle A représente un groupe hydrocarboné aromatique substitué ou non substitué ou un groupe hétérocyclique aromatique substitué ou non substitué, et X représente un groupe choisi parmi les formules (2) à (5) suivantes: 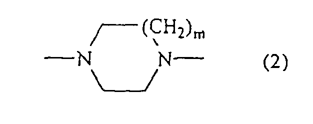 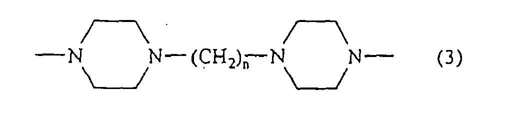 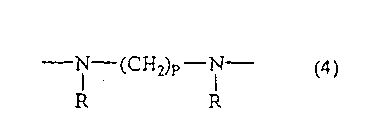 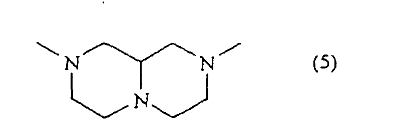 où dans la formule (2), m représente l'entier 1 ou 2 ; dans la formule (3), n représente un entier de 1 à 6 ; dans la formule (4), R représente un atome d'hydrogène ou un groupe alkyle inférieur et p représente un entier de 1 à 6, le fragment alkyle inférieur étant un groupe alkyle linéaire, ramifié ou cyclique ayant de 1 à 6 atome(s) de carbone.

2. Dérivé bis(5-aryl-2-pyridyle) ou sel de celui-ci selon la revendication 1, pour lequel dans la formule (1), A est un groupe phényle substitué ou non substitué, un groupe pyridyle substitué ou non substitué, un groupe thiényle substitué ou non substitué, un groupe furyle substitué ou non substitué, un groupe benzofuryle substitué ou non substitué, ou un groupe benzothiényle substitué ou non substitué.

3. Dérivé bis (5-aryl-2-pyridyle) ou sel de celui-ci selon la revendication 1, pour lequel dans la formule (1), A est un groupe phényle substitué ou non substitué.

4. Dérivé bis(5-aryl-2-pyridyle) ou sel de celui-ci selon la revendication 1, pour lequel ledit groupe hydrocarboné aromatique ou ledit groupe hétérocyclique aromatique est substitué par 1 à 3 substituant(s) choisi(s) parmi des groupes alkyle inférieurs, des groupes halogénoalkyle inférieurs, des groupes hydroxyalkyle inférieurs, des groupes (alkoxy inférieur)alkyle inférieurs, des groupes alkoxy inférieurs, des atomes d'halogène, des groupes hydroxy, des groupes cyano, des groupes (alkyl inférieurs)thio, des groupes amino, des groupes mono- ou di-(alkyl inférieur)amino, des groupes (alkyl inférieur)sulfonylamino, des groupes formyle, des groupes carboxyle, des groupes (alkoxy inférieur)carbonyle, des groupes alcanoyle inférieurs, des groupes pyrrolidinyle, et des groupes alkylènedioxy ; dans lesquels les fragments alkyle inférieurs sont des groupes alkyle linéaires, ramifiés ou cycliques ayant 1 à 6 atome(s) de carbone.

5. Dérivé bis(5-aryl-2-pyridyle) ou sel de celui-ci selon la revendication 1, pour lequel dans la formule (1), X est le groupe représenté par la formule (2).

6. Médicament comprenant, en tant qu'ingrédient actif, un dérivé bis (5-aryl-2-pyridyle) ou un sel de celui-ci, selon l'une quelconque des revendications 1-5.

7. Médicament selon la revendication 6, qui est un produit préventif ou thérapeutique pour une maladie immune allergique.

8. Médicament selon la revendication 6, qui est un produit préventif ou thérapeutique pour l'asthme, la dermatite atopique, la rhinite allergique, la colite inflammatoire, une maladie de peau par contact ou une maladie allergique des yeux.

9. Utilisation du dérivé bis(5-aryl-2-pyridyle) ou d'un sel de celui-ci selon l'une quelconque des revendications 1-5 pour la fabrication d'un médicament pour prévenir ou traiter une maladie immune allergique.

10. Utilisation selon la revendication 9, dans laquelle le médicament est un produit préventif ou thérapeutique pour l'asthme, la dermatite atopique, la rhinite allergique, la colite inflammatoire, des maladies de peau par contact ou des maladies allergiques des yeux.

11. Inhibiteur de la production d'anticorps IgE qui est un dérivé bis (5-aryl-2-pyridyle) ou un sel de celui-ci selon l'une quelconque des revendications 1-5.

12. Composition médicamenteuse comprenant un dérivé bis(5-aryl-2-pyridyle) ou un sel de celui-ci selon l'une quelconque des revendications 1-5 et un véhicule pharmacologiquement acceptable.
